# EUROPÄISCHE PATENTSCHRIFT

(11) **EP 2 004 700 B1**
(45) Veröffentlichungstag und Bekanntmachung des Hinweises auf die Patenterteilung: **13.03.2013**
(21) Anmeldenummer: 07727259.9
(22) Anmeldetag: 23.03.2007
(51) Int. Cl.: C08F 2/04, C08F 220/10, C08F 220/54, A61K 8/00

(54) **VERFAHREN ZUR HERSTELLUNG VON ACRYLATCOPOLYMEREN**
PROCESS FOR PREPARING ACRYLATE COPOLYMERS
PROCEDE DE FABRICATION DE COPOLYMERES D'ACRYLATE

(30) Priorität: 31.03.2006 EP 06112121
(43) Veröffentlichungstag der Anmeldung: 24.12.2008
(73) Patentinhaber: BASF SE, 67056 Ludwigshafen (DE)
(72) Erfinder: NGUYEN KIM, Son, 69502 Hemsbach (DE); LAUBENDER, Matthias, 67105 Schifferstadt (DE); PIEROBON, Marianna, 67063 Ludwigshafen (DE); WINTER, Gabi, Shanghai 201204 (CN)
(74) Vertreter: Reinhardt, Jürgen
(86) Internationale Anmeldenummer: PCT/EP2007/052785
(87) Internationale Veröffentlichungsnummer: WO 2007/113129

(56) Entgegenhaltungen:
- EP-A2- 0 037 378
- GB-A- 864 311
- US-A- 5 632 976

## Beschreibung

Die vorliegende Erfindung betrifft ein Verfahren zur Herstellung von Polymeren durch radikalische Polymerisation in Lösung, das dadurch gekennzeichnet ist, dass als Polymerisationsinitiator ein ethanollöslicher Initiator verwendet wird und die Lösungspolymerisation in einem alkoholischen Lösungsmittel durchgeführt wird, welches 5 bis 50 Gew.-% Wasser enthält.

Aus dem Stand der Technik sind zahlreiche Verfahren zur Herstellung wasserunlöslicher Polymere für die Haarkosmetik bekannt. Dazu gehören beispielsweise die Lösungs-, Fällungs-, Suspensions- oder Emulsionspolymerisation.

EP-A 0 694 565 beschreibt ein Verfahren zur homogenen Polymerisation wasserunlöslicher Polymere, die mehr als 50 Gew.-% Monomere, ausgewählt aus der aus C1-C18-Alkylacrylat oder -Methacrylatestern, N-substituierten Acryl- oder Methacrylamiden und Mischungen derselben bestehenden Gruppe, enthalten, in im wesentlichen nicht-wässrigen organischen Lösungsmitteln, dadurch gekennzeichnet, dass als Polymerisationsinitiator ein wasserlöslicher Initiator verwendet wird, der in einer zum Lösen des Initiators ausreichenden Wassermenge gelöst ist, wobei die Wassermenge 25 Gew.-% der Gesamtlösung nicht übersteigt und wobei das erhaltene Polymer durch geringere Restmonomergehalte gekennzeichnet ist als sie unter Verwendung äquivalenter Mengen wasserunlöslicher Initiatoren erhalten werden.
Weiterhin wird beschrieben, dass die Verwendung von organischen Initiatoren zu Zersetzungsprodukten mit unerwünschten Eigenschaften wie Toxizität und/oder schlechtem Geruch führt.

WO 94/24986 beschreibt die Herstellung von Haarfestigerpolymeren auf Basis von Acrylsäure und Acrylsäureestern durch Lösungspolymerisation in Ethanol. Die Polymerisation in zwischen 4 und 50 Gew.-% Wasser enthaltenden, alkoholischen Lösungsmitteln ist nicht beschrieben.

EP-A 0 379 082 beschreibt die Herstellung von Haarfestigerpolymeren auf Basis von Acrylsäure und Acrylsäureestern durch Lösungspolymerisation in Ethanol. Die Polymerisation in zwischen 4 und 50 Gew.-% Wasser enthaltenden, alkoholischen Lösungsmitteln ist nicht beschrieben.

Die aus dem Stand der Technik bekannten Verfahren haben häufig den Nachteil, dass die Reaktionszeiten lange sind, insbesondere bei Acrylsäure enthaltenden Polymerisaten, und dass die erhaltenen Polymerisate häufig hohe Restmonomer-Gehalte aufweisen. Zur Beseitigung der Restmonomere sind dann entweder Nachpolymerisations- und/oder aufwändige Reinigungsschritte notwendig.

Die durch Verfahren aus dem Stand der Technik erhältlichen Polymere besitzen häufig keine ausreichend hohen Molekulargewichte und weisen beispielsweise in der haarkosmetischen Anwendung nicht die gewünschte Flexibilität auf.

Die Aufgabe der vorliegende Erfindung bestand somit darin, ein für die Herstellung von wasserunlöslichen, für kosmetische Anwendungen geeigneten Polymeren verbessertes Verfahren bereitzustellen, das die Nachteile der bekannten Verfahren überwindet.

Gelöst wurde diese Aufgabe durch Text von Anspruch 1 einsetzen !

Im Rahmen der vorliegenden Erfindung umfasst der Ausdruck Alkyl geradkettige und verzweigte Alkylgruppen, die weiterhin auch substituiert sein können. Geeignete kurzkettige Alkylgruppen sind geradkettige oder verzweigte C₁-C₁₈-Alkyl-, bevorzugt C₁-C₁₂-Alkyl-, weiter bevorzugt C₁-C₈-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, Octyl etc..

### ai) C₁-C₁₈-Alkyl(meth)acrylate

Geeignete C₁-C₁₈-Alkyl(meth)acrylate sind Methyl(meth)acrylat, Methylethacrylat, Ethyl(meth)acrylat, n-Propyl(meth)acrylat, i-Propyl(meth)acrylat, n-Butyl(meth)acrylat, i-Butyl(meth)acrylat, sec- Butyl(meth)acrylat, tert.-Butyl(meth)acrylat, 2-Pentyl(meth)acrylat, 3-Pentyl(meth)acrylat, Isopentyl(meth)acrylat, Neopentyl(meth)acrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, O-leyl(meth)acrylat, Linolyl(meth)acrylat, Linolenyl(meth)acrylat, Stearyl(meth)acrylat, Lauryl(meth)acrylat, Phenoxyethyl(meth)acrylat, 4-t-Butylcyclohexylacrylat, Cyclohexyl(meth)acrylat, Ureido(meth)acrylat, Tetrahydrofurfuryl(meth)acrylat und deren Mischungen.

Die durch das erfindungsgemäße Verfahren hergestellten Polymere enthalten wenigstens 50, bevorzugt wenigstens 55, besonders bevorzugt wenigstens 60 und insbesondere wenigstens 70 Gew.-% C₁-C₁₈-Alkyl(meth)acrylate einpolymerisiert.

Die durch das erfindungsgemäße Verfahren hergestellten Polymere enthalten höchstens 95, bevorzugt höchstens 90, besonders bevorzugt höchstens 80 und insbesondere höchstens 75 Gew.-% C₁-C₁₈-Alkyl(meth)acrylate einpolymerisiert.

### Weitere Monomere b)

Die nach dem erfindungsgemäßen Verfahren erhältlichen Polymere enthalten bevorzugt noch weitere Monomere b) einpolymerisiert. Dies können alle radikalisch polymerisierbaren Verbindungen sein, die zu kosmetisch akzeptablen Copolymeren polymerisiert werden können.

Solche bevorzugten weiteren Monomere sind:
bi) anionische oder anionogene, radikalisch polymerisierbare Verbindungen unter einer anionogenen Verbindung wird eine Verbindung verstanden, die durch Deprotonierung mit Basen in die entsprechende anionische Form überführt werden kann. Bevorzugt sind die anionischen oder anionogenen Verbindungen ausgewählt aus der Gruppe bestehend aus (Meth)acrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure, Fumarsäure und Mischungen davon, wobei (Meth)acrylsäure und insbesondere Methacrylsäure und Methacrylsäure enthaltende Mischungen von anionischen oder anionogenen Verbindungen bevorzugt sind. Weiterhin sind auch Halbester von monoethylenisch ungesättigten Dicarbonsäuren mit 4 bis 10 vorzugsweise 4 bis 6 C-Atomen, z. B. von Maleinsäure wie Maleinsäuremonomethylester geeignet.
Besonders bevorzugt sind als anionische oder anionogene Verbindung Acrylsäure, Methacrylsäure, Itaconsäure und deren Mischungen.
In einer bevorzugten Ausführungsform beträgt das Gewichtsverhältnis von zur Polymerisation eingesetzter Methacrylsäure zu diesen weiteren Verbindungen, wie beispielsweise Acrylsäure, bevorzugt wenigstens 2:1, besonders bevorzugt wenigstens 2,5:1 und insbesondere wenigstens 3:1, wobei sich die Mengenangaben auf die Säureform der Verbindungen bi) beziehen.
Anionische oder anionogene Verbindungen sind auch die Salze der zuvor genannten Säuren. Dazu zählen alle Salze, die durch Umsetzung der Säuren mit Basen erhalten werden können. Dies sind insbesondere die Natrium-, Kalium-, Ammoniumsalze und die Salze aus der Umsetzung der Säuren mit kosmetisch üblichen OH-Gruppen tragenden Basen wie Triethanolamin oder 2-Amino-2-Methylpropanol (AMP).
bii) Ester von α,β-ethylenisch ungesättigten Mono- und Dicarbonsäuren mit C₂- bis C₁₂-Aminoalkoholen, welche am Aminstickstoff C₁- bis C₈-dialkyliert sind.
Geeignete Verbindungen dieser Art sind z. B. N,N-Dimethylaminomethyl-(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat, N,N-Dimethylaminocyclohexyl(meth)acrylat etc. Bevorzugt werden N,N-Dimethylaminopropylacrylat und N,N-Dimethylaminopropyl(meth)acrylat;
biii) Ester von α,β-ethylenisch ungesättigten Mono- und Dicarbonsäuren mit mehrwertigen Alkoholen, insbesondere Diolen.
Bevorzugte Verbindungen dieser Art sind Ester der Acrylsäure, Methacrylsäure oder Ethacrylsäure mit Diolen wie beispielsweise 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat, Neopentylglykolmono(meth)acrylat, 1,5-Pentandiolmono(meth)acrylat, 1,6-Hexandiolmono(meth)acrylat und Mischungen davon.
biv) Amide von α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen. Bevorzugt werden als Monomere e) N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl]acrylamid und N-[4-(dimethylamino)cyclohexyl]methacrylamid eingesetzt. Besonders bevorzugt werden N-[3-(dimethyl-amino)propyl]acrylamid und/oder N-[3-(dimethylamino)propyl]methacrylamid eingesetzt.
bv) vinyl- und allylsubstituierte heteroaromatische Verbindungen
Geeignete Verbindungen dieser Art sind beispielsweise 2- und 4-Vinylpyridin, Allylpyridin, und bevorzugt N-Vinylheteroaromaten, wie N-Vinylimidazol oder N-Vinyl-2-methylimidazol; N-Vinylimidazole der allgemeinen Formel VII, worin R¹ bis R³ für Wasserstoff, C₁-C₄-Alkyl oder Phenyl steht 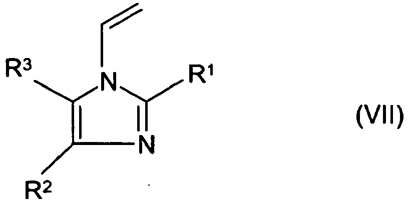 Beispiele für Verbindungen der allgemeinen Formel VII sind folgender Tabelle 1 zu entnehmen:

**Tabelle 1**

| R¹ : | R² : | R³ : |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl; Ph = Phenyl | | |

bvi) im Wesentlichen hydrophile, nichtionische Verbindungen
Bevorzugte Verbindungen dieser Art sind N-Vinylamide, N-Vinyllactame, vinyl- und allylsubstituierte heteroaromatische Verbindungen, Polyetheracrylate; bevorzugte N-Vinyllactame sind beispielsweise solche, die einen oder mehrere C₁-C₆-Alkylsubstituenten, wie Methyl, Ethyl, n-Propyl, Isopropyl, n-Butyl, sec.-Butyl, tert.-Butyl etc. aufweisen. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam und N-Vinyl-7-ethyl-2-caprolactam, wobei N-Vinylpyrrolidon und N-Vinylcaprolactam besonders bevorzugt sind.
bvii) olefinisch ungesättigte urethangruppenhaltige radikalisch polymerisierbare Verbindungen
Als Komponente bvii) wird vorteilhaft wenigstens eine olefinisch ungesättigte urethangruppenhaltige Verbindung eingesetzt. Unter olefinisch ungesättigten urethangruppenhaltigen Verbindungen werden im Rahmen der vorliegenden Erfindung Verbindungen verstanden, die wenigstens eine Urethangruppe und wenigstens eine radikalisch polymerisierbare olefinische Doppelbindung enthalten.
Als Komponente bvii) für die durch das erfindungsgemäße Verfahren erhältlichen Polymere geeignete olefinisch ungesättigte, Urethangruppen enthaltende Prepolymere sind beispielsweise in P. K. T. Oldring (Hrsg.), Chemistry and Technology of UV- and EB-Formulations for Coatings, Inks and Paints, Vol. 11, SITA Technology, London, 1991, S. 73-123 genannt, worauf hiermit in vollem Umfang Bezug genommen wird. (Poly)urethan(meth)acrylate sind dem Fachmann bekannt. Sie können erhalten werden durch Umsetzung eines Di- oder Polyisocyanates mit einem Kettenverlängerungsmittel aus der Gruppe der Diole/ Polyole und/oder Diamine/Polyamine und/oder Dithiole/Polythiole und/oder Alkanolamine und anschließende Umsetzung der restlichen freien Isocyanatgruppen mit mindestens einem Hydroxyalkyl(meth)acrylat oder Hydroxyalkylester anderer ethylenisch ungesättigter Carbonsäuren. Die Mengen an Kettenverlängerungsmittel, Di- bzw. Polyisocyanat und Hydroxyalkylester werden dabei bevorzugt so gewählt, dass
1.) das Äquivalentverhältnis der NCO-Gruppen zu den reaktiven Gruppen des Kettenverlängerungsmittels (Hydroxyl-, Amino- bzw. Mercaptylgrupppen) zwischen 3:1 und 1:2, bevorzugt bei etwa 2:1 liegt und
2.) die OH-Gruppen der Hydroxyalkylester der ethylenisch ungesättigten Carbonsäuren in stöchiometrischen Mengen in Bezug auf die noch freien Isocyanatgruppen des Präpolymeren aus Isocyanat und Kettenverlängerungsmittel vorliegen.
Es ist auch möglich, (Poly)urethan(meth)acrylate herzustellen, indem zunächst ein Teil der Isocyanatgruppen eines Di- oder Polyisocyantes mit mindestens einem Hydroxyalkylester umgesetzt wird und die restlichen Isocyanatgruppen anschließend mit einem Kettenverlängerungsmittel umgesetzt werden. Auch in diesem Fall werden die Mengen an Kettenverlängerungsmittel, Isocyanat und Hydroxyalkylester so gewählt, dass das Äquivalentverhältnis der NCO-Gruppen zu den reaktiven Gruppen des Kettenverlängerungsmittels zwischen 3:1 und 1:2, bevorzugt bei etwa 2:1 liegt und das Äquivalentverhältnis der restlichen NCO-Gruppen zu den OH-Gruppen des Hydroxyalkylesters etwa 1:1 beträgt. Selbstverständlich sind auch sämtliche Zwischenformen dieser beiden Verfahren möglich. Beispielsweise kann ein Teil der Isocyanatgruppen eines Diisocyanates zunächst mit einem Diol umgesetzt werden, anschließend kann ein weiterer Teil der Isocyanatgruppen mit dem Hydroxalkylester und im Anschluß hieran die restlichen Isocyanatgruppen mit einem Diamin umgesetzt werden. Diese verschiedenen Herstellverfahren der Polyurethan(meth)acrylate sind bekannt (z.B. aus EP-A 0 203 161) und bedürfen daher keiner detaillierten Beschreibung.
Als Komponente bvii) geeignete Urethan(meth)acrylate sind auch in der DE-A 198 38 852 S.3, Z.45 bis S.9, Z.20 beschrieben, worauf an dieser Stelle in vollem Umfang Bezug genommen wird.
Unter Urethan(meth)acrylaten werden auch Verbindungen verstanden, die
A) wenigstens eine Verbindung, die mindestens ein aktives Wasserstoffatom und mindestens eine radikalisch polymerisierbare, α,β-ethylenisch ungesättigte Doppelbindung pro Molekül enthält,
B) wenigstens ein Diisocyanat und
C) wenigstens eine Verbindung, die zwei aktive Wasserstoffatome pro Molekül enthält,
   eingebaut enthalten, und die Salze davon.

### Komponente A)

Geeignete Verbindungen A) sind z.B. die üblichen, dem Fachmann bekannten Vinyl-verbindungen, die zusätzlich wenigstens eine gegenüber Isocyanatgruppen reaktive Gruppe aufweisen, die vorzugsweise ausgewählt ist unter Hydroxylgruppen sowie primären und sekundären Aminogruppen. Dazu zählen z.B. die Ester α,β-ethylenisch ungesättigter Mono- und Dicarbonsäuren mit mindestens zweiwertigen Alkoholen.

Als α,β-ethylenisch ungesättigte Mono- und/oder Dicarbonsäuren können z. B. Acrylsäure, Methacrylsäure, Fumarsäure, Maleinsäure, Crotonsäure, Itaconsäure etc. und Gemische davon eingesetzt werden.

Geeignete Alkohole sind übliche Diole, Triole und Polyole, z.B. 1,2-Ethandiol, 1,3-Propandiol, 1,4-Butandiol, 1,5-Pentandiol, 1,6-Hexandiol, 1,10-Decandiol, Diethylenglykol, 2,2,4-Trimethylpentandiol-1,5, 2,2-Dimethylpropandiol-1,3, 1,4-Dimethylolcyclohexan, 1,6-Dimethylolcyclohexan, Glycerin, Trimethylolpropan, Erythrit, Pentaerythrit und Sorbit.

Bei den Verbindungen A) handelt es sich beispielsweise um Hydroxymethyl(meth)acrylat, Hydroxyethylethacrylat, 2-Hydroxyethyl(meth)acrylat, 2-Hydroxypropyl(meth)acrylat, 3-Hydroxypropyl(meth)acrylat, 3-Hydroxybutyl(meth)acrylat, 4-Hydroxybutyl(meth)acrylat, 6-Hydroxyhexyl(meth)acrylat, 3-Hydroxy-2-ethylhexyl(meth)acrylat sowie um Di(meth)acrylsäureester des 1,1,1-Trimethylolpropans oder des Glycerins.

Geeignete Verbindungen A) sind weiterhin die Ester und Amide der zuvor genannten α,β-ethylenisch ungesättigten Mono- und Dicarbonsäuren mit C₂- bis C₁₂-Aminoalkoholen, die eine primäre oder sekundäre Aminogruppe aufweisen. Dazu zählen Aminoalkylacrylate und Aminoalkylmethacrylate und deren N-Monoalkylderivate, die z. B. einen N-C₁- bis C₈-Monoalkylrest tragen, wie Aminomethyl(meth)acrylat, Aminoethyl(meth)acrylat, N-Methylaminomethyl(meth)acrylat, N-Ethylaminomethyl(meth)acrylat, N-Ethylaminoethyl(meth)acrylat, N-(n-propyl)aminomethyl(meth)acrylat, N-Isopropylaminomethyl(meth)acrylat und bevorzugt tert.-Butylaminoethylacrylat und tert.-Butylaminoethylmethacrylat. Dazu zählen weiterhin N-(Hydroxy-C₁- bis C₁₂-alkyl)(meth)acrylamide, wie N-Hydroxymethyl(meth)acrylamid, N-Hydroxyethyl(meth)acrylamid etc.

Geeignete Verbindungen A) sind auch die Amide der zuvor genannten α,β-ethylenisch ungesättigten Mono- und Dicarbonsäuren mit Di- und Polyaminen, die mindestens zwei primäre oder zwei sekundäre oder eine primäre und eine sekundäre Aminogruppe(n) aufweisen. Dazu zählen z. B. die entsprechenden Amide der Acrylsäure und Methacrylsäure, wie Aminomethyl(meth)acrylamid, Aminoethyl(meth)acrylamid, Aminopropyl(meth)acrylamid, Amino-n-butyl(meth)acrylamid, Methylaminoethyl(meth)acrylamid, Ethylaminoethyl(meth)acrylamid, Methylaminopropyl(meth)acrylamid, Ethylaminopropyl(meth)acrylamid oder Methylamino-n-butyl(meth)acrylamid.

Geeignete Verbindungen A) sind auch die Reaktionsprodukte von Epoxidverbindungen, die mindestens eine Epoxidgruppe aufweisen, mit den zuvor genannten α,β-ethylenisch ungesättigten Mono- und/oder Dicarbonsäuren und deren Anhydriden. Geeignete Epoxidverbindungen sind z. B. Glycidylether, wie Bisphenol-A-diglycidylether, Resorcindiglycidylether, 1,3-Propandioldiglycidylether, 1,4-Butandioldiglycidylether, 1,5-Pentandioldiglycidylether oder 1,6-Hexandioldiglycidylether.

### Komponente B)

Bei der Komponente B) handelt es sich um übliche aliphatische, cycloaliphatische und/oder aromatische Diisocyanate, wie Tetramethylendiisocyanat, Hexamethylendiisocyanat, Methylendiphenyldüsocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon. Bevorzugt handelt es sich bei der Komponente B) um Hexamethylendiisocyanat, Isophorondiisocyanat, o- und m-Xylylendiisocyanat, Dicyclohexylmethandiisocyanat und Mischungen davon. Gewünschtenfalls können bis zu 3 Mol-% der genannten Verbindungen durch Triisocyanate ersetzt sein.

### Komponente C)

Geeignete Verbindungen C) sind z. B. Diole, Diamine, Aminoalkohole und Mischungen davon. Gewünschtenfalls können bis zu 3 Mol-% der genannten Verbindungen durch Triole oder Triamine ersetzt sein.

Geeignete Diole C) sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt.

Geeignete Aminoalkohole C) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol oder 4-Methyl-4-aminopentan-2-ol.

Geeignete Diamine C) sind z. B. Ethylendiamin, Propylendiamin, 1,4-Diaminobutan, 1,5-Diaminopentan und 1,6-Diaminohexan.

Bevorzugte Verbindungen C) sind Polymerisate mit einem zahlenmittleren Molekulargewicht im Bereich von etwa 300 bis 5000, bevorzugt etwa 400 bis 4000, insbesondere 500 bis 3000. Dazu zählen z. B. Polyesterdiole, Polyetherole, α,ω-Diaminopolyether und Mischungen davon. Vorzugsweise werden ethergruppenhaltige Polymerisate eingesetzt.

Bei den Polyetherolen C) handelt es sich vorzugsweise um Polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten.

Geeignete α,ω-Diaminopolyether C) sind z. B. durch Aminierung von Polyalkylenoxiden mit Ammoniak herstellbar.

Geeignete Polytetrahydrofurane C) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Geeignete Polyesterdiole C) weisen vorzugsweise ein zahlenmittleres Molekulargewicht im Bereich von etwa 400 bis 5000, bevorzugt 500 bis 3000, insbesondere 600 bis 2000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure.

Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, sowie Poly(meth)acrylatdiole der Formel 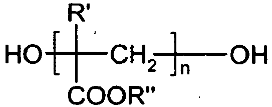 worin R' für H oder CH₃ steht und R" für C₁-C₁₈-Alkyl (insbesondere C₁-C₁₂- oder C₁-C₈-Alkyl) steht, die eine Molmasse von bis zu etwa 3000 aufweisen. Derartige Diole sind auf übliche Weise herstellbar und im Handel erhältlich (Tegomer^{®}-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% und bevorzugt 50 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/ Dimethylolcyctohexan.

Die Verbindungen C) können einzeln oder als Mischungen eingesetzt werden. Weitere mögliche Urethan(meth)acrylate sind der DE-A 198 38 852 S.5, Z.40 bis S.9, Z.20 zu entnehmen, worauf hiermit in vollem Umfang Bezug genommen wird. Insbesondere geeignet sind die in der DE-A 198 38 852, Tabelle 1 beschriebenen Urethandiacrylate.

Als geeignete Komponenten bvii) seien weiter genannt:
bviia) Reaktionsprodukte der Umsetzung von Hydroxy(Meth)acrylaten mit Diolen und/oder OH-terminierten Polyolen und/oder OH-terminierten Polyestern und/oder Diaminen und Diisocyanaten. Solche difunktionellen Urethanacrylat-Oligomere und ihre Herstellung sind beispielsweise beschrieben in WO 97/00664, S.5,Z.17 bis S.6, Z.8 und den entsprechenden Beispielen, worauf hier vollumfänglich Bezug genommen wird.
bviib) Carbamoyloxycarboxylate der allgemeinen Formel **I** 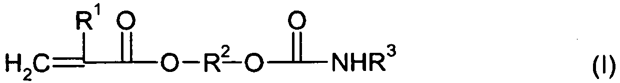 wobei
   R¹ H, Halogen oder C1-C8-Alkyl,
   R² gegebenenfalls subsituiertes C₁-C₁₂-Alkylen, -Arylen, -Alkylarylen oder - Arylalkylen, Polyoxyalkylen,
   R³ C₁-C₈-Alkyl bedeuten.
   Solche Carbamoyloxycarboxylate der allgemeinen Formel I sind in US 3,479,328 und US 3,674,838 offenbart, worauf hiermit in vollem Umfang Bezug genommen wird.
bviic) die in der GB 1 443 715 offenbarten Divinylurethane der allgemeinen Formel 11 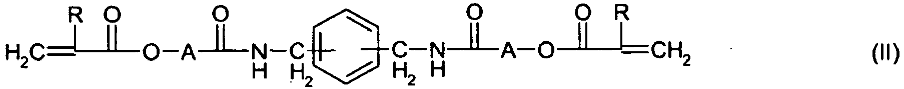 wobei
   R H oder Methyl
   A (Poly)Alkylenoxy bedeuten
   und Vinylurethane der allgemeinen Formel **III** 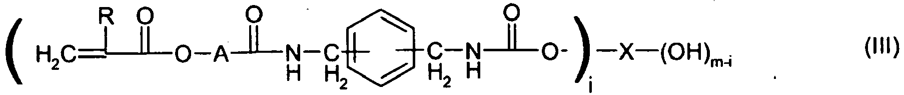 wobei R und A wie in Formel II und X und n wie in GB 1 443 715 S.2, Z.9-13 definiert. Die ebenfalls in der GB 1 443 715, auf die hiermit in vollem Umfang Bezug genommen wird, beschriebenen Vinylurethane sind weitere mögliche Komponenten c) der durch das erfindungsgemäße Verfahren herstellbaren Polymere.
bviid) die in der EP-A 0 036 813, auf die hiermit in vollem Umfang Bezug genommen wird, beschriebenen N-substituierten Carbamoyloxyalkylenoxyalkyl(meth)acrylate der allgemeinen Formel IV 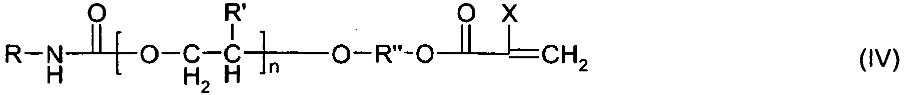 wobei R, R', R" und X wie in EP-A 0 036 813, S.2, Z.13-28 definiert und n eine ganze Zahl von 0 bis 20, bevorzugt von 1 bis 6 und besonders bevorzugt von 1 bis 4 bedeutet.
bviie) die aus der DE-A-4 007 146, auf die hiermit in vollem Umfang Bezug genommen wird, bekannten Urethanacrylat-Verbindungen, die erhältlich sind durch Umsetzung von Polyisocyanaten mit Hydroxyalkylacrylaten, gefolgt von einer Umsetzung mit primären oder sekundären Aminen.
bviif) Produkte der Umsetzung von Isocyanaten mit Polyolen und Hydroxyalkylacrylaten wie beispielsweise in DE 27 26 041 A, US 4,260,703 und US 4,481,093 beschrieben und Produkte der Umsetzung von Isocyanaten mit Hydroxyalkylacrylaten wie in JP 63297369 und JP 59157112 beschrieben, worauf hiermit in vollem Umfang Bezug genommen wird.
bviig) die in der EP-A 0 903 363, auf die hier hiermit in vollem Umfang Bezug genommen wird, beschriebenen Urethangruppen enthaltende Prepolymere, die durch ein Verfahren herstellbar sind, wobei man eine Isocyanatgruppen enthaltende Komponente A mit einer OH-Gruppen enthaltenden Komponente B umsetzt, wobei die Komponente A wenigstens eine trifunktionelle Isocyanatverbindung A1 und gegebenenfalls eine oder mehrere difunktionelle Isocyanatverbindungen A2 umfasst und die OH-Gruppen enthaltende Komponente B wenigstens eine olefinisch ungesättigte Verbindung B1 mit wenigstens einer reaktiven OH-Gruppe und gegebenenfalls davon verschiedene OH-Gruppen enthaltende Verbindungen B2 umfasst, wobei entweder die Komponente A zwei verschiedene Isocyanatverbindungen A1 oder eine Isocyanatverbindung A1 und wenigstens eine Isocyanatverbindung A2 umfasst oder die Komponente B wenigstens zwei verschiedene Verbindungen B2 umfasst.
bviih) Polyurethanpolymerisate, welche A) 40 bis 80 Gew.-%, bezogen auf das Gesamtgewicht der Komponenten A) bis F), wenigstens eines hydroxyl gruppenhaltigen Präpolymers mit mindestens einer radikalisch oder photochemisch polymerisierbaren α,β-ethylenisch ungesättigten Doppelbindung, wobei das Präpolymer
   A) ein Reaktionsprodukt oder eine Mischung aus a) wenigstens einem Polyesteractylat und/oder Polyetheractylat und/oder Polyurethanacrylat und b) wenigstens einem Epoxyactylat ist,
   B) 0,1 bis 20 Gew.-%, bezogen auf das Gesamtgewicht der Komponenten A) bis F), wenigstens einer Verbindung mit mindestens einer gegenüber Isocyanatgruppen reaktiven Hydroxyl- und/oder primären oder sekundären Aminogruppe und zusätzlich wenigstens einer polaren funktionellen Gruppe,
   C) 0,1 bis 10 Gew.-%, bezogen auf das Gesamtgewicht der Komponenten A) bis F), wenigstens einer Verbindung, ausgewählt unter Diaminen, Polyaminen und Mischungen davon,
   D) 0 bis 20 Gew.%, bezogen auf das Gesamtgewicht der Komponenten A) bis F), wenigstens einer weiteren von A), B), C) und E) verschiedenen Verbindung mit mindestens zwei gegenüber Isocyanatgruppen reaktiven Gruppen, wobei es sich um Hydroxylgruppen und Mischungen von Hydroxylgruppen und/oder primären oder sekundären Aminogruppen handelt,
   E) 0 bis 20 Gew.-%, bezogen auf das Gesamtgewicht der Komponenten A) bis F), wenigstens einer Verbindung mit einer gegenüber Isocyanatgruppen reaktiven Gruppe,
   F) 10 bis 50 Gew.-%, bezogen auf das Gesamtgewicht der Komponenten A) bis F), wenigstens eines Polyisocyanates einpolymerisiert enthält, und die Salze davon, die dadurch gekennzeichnet sind, dass die Summe der Hydroxylzahlen der Komponenten A) und D) in einem Bereich von 121 bis 300 mg KOH/g liegt.
   Diese Polyurethanpolymerisate sind in der EP-A 0 942 022 beschrieben, auf die hier hiermit in vollem Umfang Bezug genommen wird.
bviij) die in der EP-A 1 002 818 (auf die hier hiermit in vollem Umfang Bezug genommen wird) beschriebenen Umsetzungsprodukte von
   a) Isocyanat-Trimeren(gemische) auf der Basis aliphatischer oder cycloaliphatischer Diisocyanate, welche zu bis 100 mol-% aus Verbindungen vom Iminooxadiazindion-Strukturtyp der Formel A bestehen, 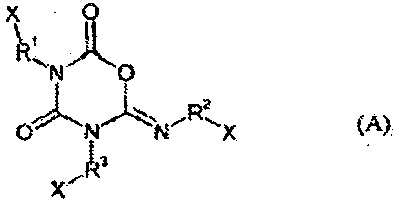 in welcher R¹, R² und R³ unabhängig voneinander für gegebenenfalls verzweigte C4-C20-(Cyclo)Alkylen stehen, und
      X für gleiche oder verschiedene Reste von Isocyanat oder von Isocyanat-Folgeprodukten, welche vom Iminooxadiazindion-, Isocyanurat-, Uretdion-, Urethan-, Allophanat-, Biuret- oder Oxadiazintrion-Strukturtyp steht und N-ständig die obengenannten Reste R¹, R² und R³ tragen, mit
   b) einer Alkoholkomponente, welche mindestens einen einwertig Hydroxy-funktionellen gegebenenfalls verzweigten C₁-C₁₂-Alkyl-Ester der (Meth)acrylsäure enthält.
bviik) die Allylgruppen enthaltenden Polyurethane der allgemeinen Formel V, wie in der WO 01/72862, auf die hiermit in vollem Umfang Bezug genommen wird, offenbart:

   R¹-[NHCO(OR)_{y} (OCH₂CH=CH₂)ₘ]ₙ (V)

   Die Bedeutungen von R, R¹**,** y, m und n sind in der WO 01/72862 auf S.3, Z.29 bis S.4, Z.10 beschrieben.
bviil) die in der WO 04/050888, auf die hiermit in vollem Umfang Bezug genommen wird, beschriebenen urethangruppenhaltigen (Meth)acrylsäureester, die herstellbar sind durch die Umsetzung eines urethangruppenhaltigen Alkohols mit (Meth)acrylsäure oder einem Ester von (Meth)acrylsäure mit einem gesättigten Alkohol und gegebenenfalls Aufreinigung des Reaktionsgemisches, wobei man die Umsetzung in Gegenwart eines Enzyms (E) durchführt.
bviim) die in der WO 98/06783, worauf an dieser Stelle in vollem Umfang Bezug genommen wird, insbesondere auf S.1, Z.22 bis S.2, Z.6 beschriebenen Urethan(meth)-acrylat-Oligomere.
bviin) die in der DE 44 34 554 A1, worauf an dieser Stelle in vollem Umfang Bezug genommen wird, insbesondere auf S.2, Z.42 bis S.4, Z.27 beschriebenen Polyurethane.
bviio) die in der WO 04/067599, auf die hiermit in vollem Umfang Bezug genommen wird, insbesondere auf S.1 0, Z.24 bis S.12, Z.13 beschriebenen Urethan-(Meth)acrylat-Oligomere.
bviip) die in der US 5240835, worauf an dieser Stelle in vollem Umfang Bezug genommen wird, beschriebenen Urethanacrylate, die herstellbar sind durch die Umesterung von Alkylacrylaten mit Alkoholen unter Katalyse eines Biokatalysators aus Corynebacterium oxydans.
bviiq) die in der WO 04/052843, worauf an dieser Stelle in vollem Umfang Bezug genommen wird, beschriebenen Carbamoyloxy(meth)acrylate, die herstellbar sind durch ein Verfahren, wie es auf S.3, Z.34 bis S.10, Z.28 der WO 04/052843 beschrieben ist.
bviir) die Carbamyloxy(meth)acrylate, die beschrieben sind in der WO 94/25537 S.8, Z.29 bis S.9, Z.32, worauf an dieser Stelle in vollem Umfang Bezug genommen wird.
bviis) die in der DE-A 102 46 112, worauf hiermit in vollem Umfang Bezug genommen wird, beschriebenen Polyisocyanatfolgeprodukte enthaltend mindestens eine Allophanatgruppe, die an dem über zwei Einfachbindungen gebundenen Sauerstoffatom der Allophanatgruppe mindestens eine Acrylat-, Methacrylat- oder Vinylether-Doppelbindung trägt, dadurch gekennzeichnet, dass ein Polyisocyanat oder Polyisocyanatfolgeprodukt enthaltend mindestens eine Oxadiazintriongruppe mit einem Acrylat-, Methacrylat- oder Vinylether-Doppelbindung enthaltenden Alkohol bei Temperaturen zwischen -20 bis 100°C reagiert.
bviit) Die WO 00/39183, worauf hiermit in vollem Umfang Bezug genommen wird, beschreibt Verbindungen mit Isocyanatgruppen oder verkappten Isocyanatgruppen, Allophanatgruppen und radikalisch polymerisierbaren C-C-Doppelbindungen, wobei die C-C-Doppelbindungen durch eine direkt daran gebundene Carbonylgruppe oder ein O-Atom in Etherfunktion aktiviert sind (Aktivierte Doppelbindungen), abgeleitet von Polyisocyanaten und Alkoholen A, die neben der Alkoholgruppe noch eine Aktivierte Doppelbindung tragen.
   Diese Verbindungen werden bevorzugt mit Alkoholen ROH, die nur eine OH-Gruppe tragen oder mit Aminen RNH₂ oder RR'NH in mindestens der Menge umgesetzt, die ausreicht, um alle Isocyanatgruppen und verkappten Isocyanatgruppen in Urethan- oder Harnstoffgruppen zu überführen.
   Dabei bedeuten R und R' unabhängig voneinander C₁-C₁₂-Alkyl, -Aryl, -Alkylaryl oder - Arylalkyl, Polyoxyalkylen, wobei die Reste gegebenenfalls mit Hydroxylgruppen funktionalisiert sein können.
   Bevorzugte Alkohole für diese Umsetzung sind C₁-C₁₂-, insbesondere C₁-C₄-Alkanole wie beispielsweise Methanol, Ethanol, n-Propanol, i-Propanol, n-Butanol, i-Butanol, sek-Butanol, tert.-Butanol.
   Bevorzugte Amine für diese Umsetzung sind C₁-C₁₂-, insbesondere C₁-C₄-(Di)Alkylamine, (Di)Alkanolamine, Alkylalkanolamine wie beispielsweise Ethylamin, Butylamin, Diethylamin, Ethanolamin, Diethanolamin, 2-Amino-2-Methylpropanol.
   Es werden auf diese Weise beispielsweise Verbindungen der folgenden allgemeinen Formeln erhalten:
   Aus der Umsetzung mit Alkoholen: 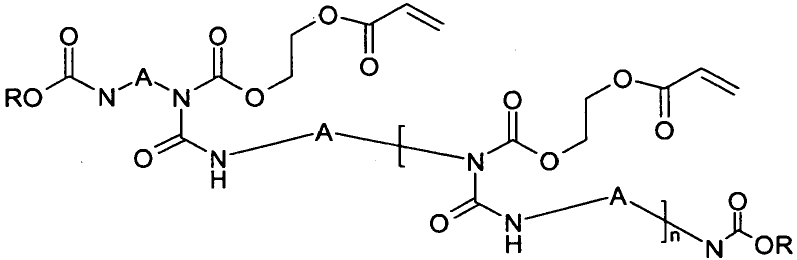
   Aus der Umsetzung mit Aminen: 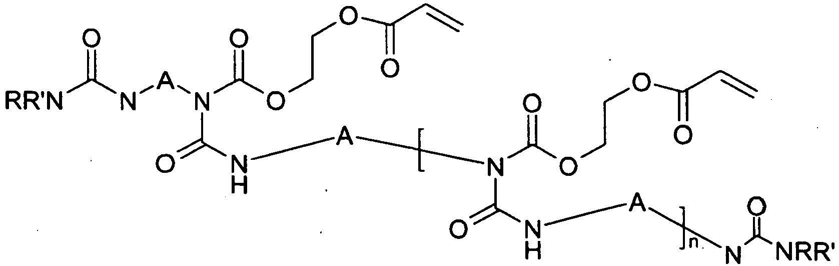 wobei
      R C₁-C₁₂-Alkyl, -Aryl, -Alkylaryl oder -Arylalkyl, Polyoxyalkylen, gegebenenfalls mit Hydroxylgruppen funktionalisiert
      R' H, C₁-C₁₂-Alkyl, -Aryl, -Alkylaryl oder -Arylalkyl, Polyoxyalkylen, gegebenenfalls mit Hydroxylgruppen funktionalisiert
      n 0 bis 10, bevorzugt 0 bis 5, besonders bevorzugt 0 bis 2
      A C₁-C₁₂-Alkylen, -Arylen, -Alkylarylen oder -Arylalkylen, Polyoxyalkylen bedeuten und Mischungen davon.

   Selbstverständlich können auch die entsprechenden Methacrylat-Derivate dieser Verbindungen als Komponente c) eingesetzt werden.
   Geeignete Komponenten c) sind beispielsweise auch
bviiu) N-butyl-2-hydroxyethyl-carbamate (CAS 63225-53-6) der Formel 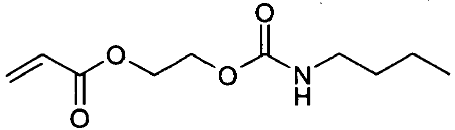 (wie beispielsweise als Ebecryl^{®}CL 1039 (UCB) kommerziell erhältlich) und das entsprechende Methacrylsäure-Derivat,
bviiv) N-methyl-2-hydroxyethyl-carbamate (CAS 52607-81-5) der Formel 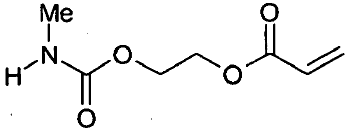 und das entsprechende Methacrylsäure-Derivat,
bviiw) eine der oder eine Mischung der beiden Komponenten der folgenden Formeln (die Mischung wird hierin als Monomer C22 bezeichnet (siehe auch die Beispiele)): 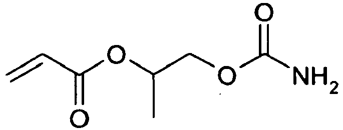 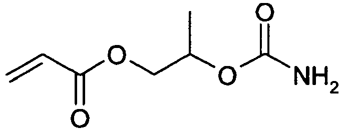 und die entsprechenden Methacrylsäure-Derivate,
bviix) eine der oder eine Mischung der beiden Komponenten der folgenden Formeln 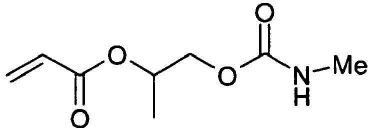 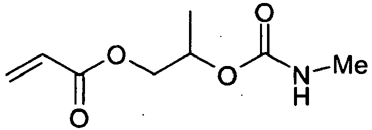 und die entsprechenden Methacrylsäure-Derivate,
bviiy) Verbindung der folgenden Formel 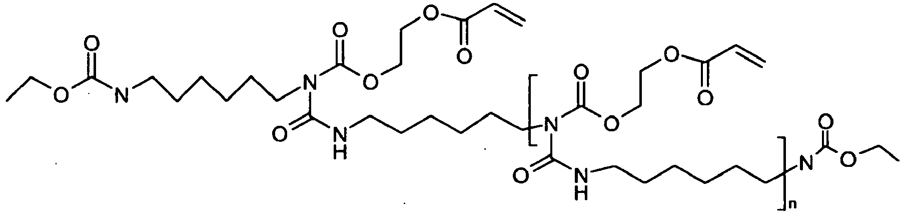 wobei n 0 bis 10, bevorzugt 0 bis 4, besonders bevorzugt 0 bis 2 bedeutet,
   und die entsprechenden Methacrylsäure-Derivate.
bvii x) Diurethandimethacrylat 7,7,9-(bzw. 7,9,9-)Trimethyl-4,13-dioxo-3,14-dioxa-5,12-diaza-hexadecan-1,16-diol-dimethacrylat (CAS 72869-86-4), das beispielsweise als PLEX^{®}6661-O (Degussa) kommerziell erhältlich ist.

Als Komponente bvii) geeignete Polyurethan(meth)acrylate wie Polyurethan-mono-, - di-, -tri-, -tetra, -penta oder -hexa- (meth)acrylate sind kommerziell erhältlich unter den Marken Laromer^{®} (BASF), Photomer^{®} (Cognis), Sartomer^{®} (Sartomer) oder Ebecryl^{®} (UCB).

Sie können in reiner Form (ohne Verdünnungsmittel), als Lösungen in Lösungsmitteln wie Ethanol oder Butylacetat oder als Lösungen in reaktiven Verdünnungsmitteln (wie beispielweise Tripropylenglykoldiacrylat (TPGDA), Hexandioldiacrylat (HDDA), Dipropylenglykoldiacrylat (DPGDA), Trimethylolpropanformalmonoacrylat (Laromer^{®}LR 8887), Trimethylolpropantriacrylat (TMPTA), propoxyliertes Glyceryltriacrylat (GPTA), Ethoxyliertes Trimethylolpropantriacrylat (EO3TMPTA), Ethoxyethoxyethylacrylat (E-OEOEA), PEG 400 diacrylat (PEG400DA), Isobornylacrylat (IBOA), Propoxyliertes Neopentylglycoldiacrylat (P02NPGDA), 2-Phenoxyethylacrylat (POEA), Butandioldiacrylat (BDDA), Butandiolacrylat (BDMA), Dihydrodicyclopentadienylacrylat (DCPA), Triethylenglycoldivinylether, Ethyldigycolacrylat (EDGA), Laurylacrylat (LA), 4-t-butylcyclohexylacrylat (TBCH) oder als wässrige Emulsionen eingesetzt werden. Solche Polyurethan(meth)acrylate sind:
Laromer^{®}-Typen Laromer-Typen LR 8949, LR 9005, LR 8983, UA 19 T, UA 9030V, UA 9028V, UA 9029V, UA 9033V, UA 9031V und LR 8987,
Photomer^{®}-Typen 6891, 6892, 6893-20R, 6572, 6010, 6019, 6184, 6210, 6217, 6230, 6363 und 6008
Sartomer^{®}CN-Typen wie beispielsweise
die aliphatischen Urethanacrylate CN 934 CN 934X50, CN 944B85, CN 945A60, CN 945B85, CN 953B70, CN 961 E75, CN 961 H81, CN 962, CN 963A80, CN 963B80, CN 963E75, CN 963E80, CN 963J85, CN 964, CN 964A85, CN 964B85, CN 964H90, CN 964E75, CN 965, CN 965A80, CN 966A80, CN 966B85, CN 966H90, CN 966180, CN 966J75, CN 966R60, CN 968, CN 982E75, CN 982P90, CN 983, CN 983B88, CN 984, CN 985B88
und die aromatischen Urethanacrylate CN 970A60, CN 970E60, CN 970H75, CN 971A80, CN 972, CN 973A80, CN 973H85, CN 973J75, CN 975, CN 977C70, CN 978, CN 980, CN 980M50, CN 981, CN 981A75, CN 981 B88, CN 982A75, CN 982B88 Ebecryl^{®}-Typen wie beispielsweise 220, 230, 244, 264, 265, 270.

Als Verbindung bvii) sind besonders bevorzugt Carbamoyloxycarboxylate der allgemeinen Formel VIII 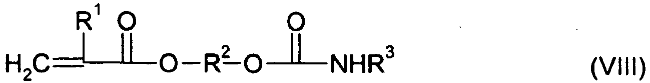 wobei
R¹ H, Halogen, C₁-C₈-Alkyl, bevorzugt H oder Methyl,
R² gegebenenfalls subsituiertes C₁-C₁₂-Alkylen, -Arylen, -Alkylarylen oder - Arylalkylen, gegebenenfalls hydroxy-substituiertes Polyoxyalkylen,
R³ H, C₁-C₈-Alkyl bedeuten.
Generell sind solche Verbindungen bvii) bevorzugt, die höchstens 4, bevorzugt höchstens 3 und besonders bevorzugt höchstens 2 radikalische polymerisierbare Doppelbindungen pro Molekül enthalten.

Die durch das erfindungsgemäße Verfahren erhältlichen Polymere enthalten 0-30, bevorzugt 0,1-20, besonders bevorzugt 0,5-10 und am meisten bevorzugt 0,5-5 Gew.-% der Verbindung iii) einpolymerisiert.

### bviii) im Wesentlichen hydrophobe, nichtionische Verbindungen

Bevorzugt Verbindungen dieser Art sind beispielsweise die Ester von Vinylalkohol oder Allylalkohol mit C₁-C₃₀-Monocarbonsäuren, Vinylether, Vinylaromaten, Vinylhalogenide, Vinylidenhalogenide, C₂-C₈-Monoolefine, nichtaromatische Kohlenwasserstoffe mit mindestens 2 konjugierten Doppelbindungen und Mischungen davon; geeignete Beispiele sind Vinylformiat, Vinylacetat, Vinylpropionat, Vinyl-n-butyrat, Vinylstearat, Vinyllaurat, Styrol, α-Methylstyrol, o-Chlorstyrol, Acrylnitril, Methacrylnitril, Vinyltoluole, Vinylchlorid, Vinylidenchlorid, Vinylfluorid, Vinylidenfluorid, Ethylen, Propylen, Isobuten, Butadien, Isopren, Chloropren, Methyl-, Ethyl-, Butyl-, Dodecylvinylether und Mischungen davon.

### bix) Polyester mit wenigstens zwei radikalisch polymerisierbaren, olefinisch ungesättigten Doppelbindungen

Der Begriff Polyester ist dem Fachmann bekannt. Polyester sind Polymere mit Esterbindungen -[-CO-O-]- in der Hauptkette. Komponenten bix) gemäß dieser Erfindung sind beispielsweise Polyester(meth)acrylate, die wenigstens zwei radikalisch polymerisierbare, olefinisch ungesättigte Doppelbindungen pro Molekül enthalten. Polyester(meth)acrylate sind dem Fachmann prinzipiell bekannt. Sie sind durch verschiedene Methoden herstellbar. Beispielsweise kann (Meth)acrylsäure direkt als Säurekomponente beim Aufbau der Polyester eingesetzt werden. Daneben besteht die Möglichkeit, Hydroxyalkylester der (Meth)acrylsäure als Alkoholkomponente direkt beim Aufbau der Polyester einzusetzen. Bevorzugt werden die Polyester(meth)acrylate aber durch (Meth)acrylierung von Polyestern hergestellt. Beispielsweise können zunächst hydroxylgruppenhaltige Polyester aufgebaut werden, die dann mit Acryl- oder Methacrylsäure umgesetzt werden. Bevorzugt werden wenigstens 2 der Hydroxylgruppen pro Molekül der hydroxylgruppenhaltige Polyester mit (Meth)acrylsäure umgesetzt, so dass pro Molekül des Reaktionsproduktes wenigstens zwei radikalisch polymerisierbare, olefinisch ungesättigte Doppelbindungen enthalten sind.

Es können auch zunächst carboxylgruppenhaltige Polyester aufgebaut werden, die dann mit einem Hydroxyalkylester der Acryl- oder Methacrylsäure umgesetzt werden. Auch hier werden wenigstens zwei der Carboxylgruppen pro Molekül der carboxylgruppenhaltige Polyester mit dem Hydroxyalkylester der (Meth)acrylsäure umgesetzt, so dass pro Molekül des Reaktionsproduktes wenigstens zwei radikalisch polymerisierbare, olefinisch ungesättigte Doppelbindungen enthalten sind. Bevorzugt ist, es Gemische von Polyester(meth)acrylaten einzusetzen, die durchschnittlich mehr als 2 radikalisch polymerisierbare, olefinisch ungesättigte Doppelbindungen pro Molekül Polyester(meth)acrylat enthalten.

Als Komponente bix) geeignete Polyesteracrylate sind beispielsweise beschrieben in der EP-A 0 279 303, auf die hier vollumfänglich Bezug genommen wird (EP-A 0 279 303, S.5, Z.28-44).

Auch die DE 2 853 921 beschreibt geeignete Polyesteracrylate, nämlich solche aus aliphatischen und/oder aromatischen Dicarbonsäuren, wie Bernsteinsäure, Glutarsäure, Adipinsäure, Pimelinsäure, Korksäure, Azelainsäure, Sebacinsäure, Cyclohexandicarbonsäure, Phthalsäure, Isophthalsäure, Terephthalsäure, Maleinsäure, Fumarsäure, Itakonsäure bzw. deren Derivaten und mehrwertigen Alkoholen, wie Ethylenglykol, Polyethylenglykolen, Propylenglykol, Polypropylenglykolen, Butandiol, Hexandiol, Neopentylglykol, Hydroxypivalinsäureneopentylglykolester, Trimethylolpropan, Glycerin, Pentaerythrit und/ oder Trishydroxyethylisocyanurat sowie α,β-ethylenisch ungesättigten Monocarbonsäuren, beispielsweise Acrylsäure, Methacrylsäure, Crotonsäure, Zimtsäure und/oder Dicarbonsäurehalbester von Monoalkanolen, wie Malein-, Fumar und Itakonsäurehalbester mit C₁-C₄-Monoalkoholen, wobei Acrylsäure und Methacrylsäure bevorzugt sind.

Auch die EP-A 0 686 621, auf die hier vollumfänglich Bezug genommen wird, beschreibt geeignete Komponenten bix). Dabei handelt es sich um Umsetzungsprodukte der (Meth)acrylsäure mit einer Hydroxyverbindung. Als Hydroxyverbindungen in Betracht kommen Verbindungen mit einer oder mehreren Hydroxygruppen. Genannt sind Monoalkohole, C₂-C₆-Alkylendiole, Trimethylolpropan, Glycerin oder Pentaerythrit oder z.B. mit Ethylenoxid oder Propylenoxid alkoxylierte, Hydroxygruppen enthaltende Verbindungen.

Als Hydroxyverbindungen kommen weiterhin hydroxylgruppenhaltige Polyester in Betracht. Derartige hydroxylgruppenhaltige Polyester können z.B. in üblicher Weise durch Veresterung von Dicarbonsäuren oder Polycarbonsäuren mit Diolen oder Polyolen hergestellt werden. Die Ausgangsstoffe für solche hydroxylgruppenhaltige Polyester sind dem Fachmann bekannt. Bevorzugt können als Dicarbonsäuren Bernsteinsäure, Glutarsäure, Adipinsäure, Sebacinsäure, o-Phthalsäure, deren **I**somere und Hydrierungsprodukte sowie veresterbare Derivate, wie Anhydride, z.B. Maleinsäureanhydrid, oder Dialkylester der genannten Säuren eingesetzt werden. Als Polycarbonsäure kommt z.B. Trimellitsäure in Betracht. Zu den einzusetzenden Polyesterolen zählen auch Polycaprolactondiole und -triole, deren Herstellung dem Fachmann ebenfalls bekannt ist.

Bevorzugte Hydroxyverbindungen sind mindestens 2, insbesondere 2 bis 6 freie Hydroxylgruppen enthaltende, gesättigte Polyester, welche gegebenenfalls auch Ethergruppen enthalten können oder Polyether (als Komponente bx)) mit mindestens 2, insbesondere 2 bis 6 freien Hydroxylgruppen.

Die Komponenten bix), wie beispielsweise Polyester(meth)acrylate besitzen wenigstens 2 radikalisch polymerisierbare Doppelbindungen pro Molekül. Weiterhin bevorzugt ist es, Gemische von beispielsweise von Polyester(meth)acrylaten einzusetzen, die durchschnittlich mehr als 2 radikalisch polymerisierbare, olefinisch ungesättigte Doppelbindungen pro Molekül Polyester(meth)acrylat enthalten. Solche Gemische ergeben sich beispielsweise durch Mischen von Verbindungen mit jeweils 2 und Verbindungen mit jeweils 3 oder mehr polymerisierbaren Doppelbindungen pro Molekül. Natürlich können in den Gemischen auch Verbindungen enthalten sein, die nur eine oder keine Doppelbindung pro Molekül enthalten. Solche Verbindungen sind dann aber in derartigen Mengen enthalten, dass die durchschnittliche Zahl von polymerisierbaren Doppelbindungen pro Molekül trotzdem mehr als 2 beträgt.

### bx) Polyether, enthaltend wenigstens zwei radikalisch polymerisierbare, olefinisch ungesättigte Doppelbindungen

Der Begriff der Polyether ist dem Fachmann bekannt. Polyether sind Polymere, deren Wiederholungseinheiten durch Ether-Funktionalitäten (C-O-C) verknüpft sind. Beispiele für Polyether sind Polyalkylenglykole (Polyethylenglykole, Polypropylenglykole, Polyepichlorhydrine) als Polymere von 1,2-Epoxiden, Epoxidharze, Polytetrahydrofurane (Polytetramethylenglykole), Polyoxetane, Polyphenylenether (Polyarylether) oder Polyether(ether)keton(keton)e.

Komponenten bx) gemäß dieser Erfindung sind beispielsweise Polyether(meth)acrylate, die wenigstens 2 radikalisch polymerisierbare Doppelbindungen pro Molekül enthalten. Diese sind dem Fachmann bekannt. Sie sind durch verschiedene Methoden herstellbar. Beispielsweise können hydroxylgruppenhaltige Polyether, die mit Acrylsäure und/oder Methacrylsäure zu den Polyether(meth)acrylaten verestert werden, durch Umsetzung von zwei- und/oder mehrwertigen Alkoholen mit verschiedenen Mengen an Ethylenoxid und/oder Propylenoxid nach gut bekannten Methoden (vgl. z.B. Houben-Weyl, Band XIV, 2, Makromolekulare Stoffe II, (1963)) erhalten werden. Einsetzbar sind auch Polymerisationsprodukte des Tetrahydrofurans oder Butylenoxids.

Auch die DE 2 853 921, auf die hier vollumfänglich Bezug genommen wird, beschreibt geeignete Komponenten bx) wie beispielsweise aliphatische oder aromatisch-aliphatische Polyether, welche durch Umsetzung von zwei- und/oder mehrwertigen Alkoholen, mit verschiedenen Mengen an Ethylen-und/oder Propylenoxid erhalten werden und deren freie Hydroxylgruppen ganz oder teilweise mit ethylenisch ungesättigten Alkoholen, beispielsweise Allylalkohol, Methallylalkohol, Crotylalkohol, Zimtalkohol, verethert und/oder mit α,β-ethylenisch ungesättigten Monocarbonsäuren verestert sind.

Als Komponente bx) geeignete Polyetheracrylate sind beispielsweise auch beschrieben in der EP-A 0 279 303, worauf hier vollumfänglich Bezug genommen wird. Diese Polyetheracrylate sind erhältlich durch Reaktion von A) 1 Äquivalent eines 2-bis 6-wertigen oxalkylierten C₂-bis C₁₀-Alkohols mit B) 0,05 bis 1 Äquivalent einer 2-bis 4-wertigen C₂-bis C₁₀-Carbonsäure oder deren Anhydride und C) 0,1 bis 1,5 Äquivalenten Acrylsäure und/oder Methacrylsäure sowie Umsetzung der überschüssigen Carboxylgruppen mit der äquivalenten Menge einer Epoxidverbindung. Auch die EP-A 0 686 621, auf die hier vollumfänglich Bezug genommen wird, beschreibt geeignete Komponenten bx). Dabei handelt es sich um Umsetzungsprodukte der (Meth)acrylsäure mit einer Hydroxyverbindung. Als Hydroxyverbindungen in Betracht kommen Verbindungen mit einer oder mehreren Hydroxygruppen. Genannt seien z.B. mit Ethylenoxid oder Propylenoxid alkoxylierte, Hydroxygruppen enthaltende Verbindungen.

Bevorzugte Hydroxyverbindungen sind gesättigte Polyether mit mindestens 2, insbesondere 2 bis 6 freien Hydroxylgruppen. Als hydroxylgruppenhaltige Polyether kommen z.B. solche in Frage, welche nach bekannten Verfahren durch Umsetzung von zwei- und/oder mehrwertigen Alkoholen mitverschiedenen Mengen an Ethylenoxid und/oder Propylenoxid erhalten werden können. Bei den Ethylenglykol/Propylenglykol-Mischkondensationsprodukten kann die Umsetzung zweckmäßigerweise so gesteuert werden, daß endständig überwiegend primäre Hydroxylgruppen entstehen. Desgleichen sind auch Polymerisationsprodukte des Tetrahydrofurans oder Butylenoxids, die Hydroxylgruppen enthalten, verwendbar. Beispiele für Komponente bx) sind Polyalkylenglykol(meth)acrylate.

In einer bevorzugten Ausführungsform der Erfindung werden als Komponente bx) solche Verbindungen verwendet, deren Molekulargewicht M_{w} mindestens 200 g/mol, besonders bevorzugt mindestens 400 g/mol, ganz besonders bevorzugt mindestens 500 g/mol und am meisten bevorzugt mehr als 700 g/mol beträgt.

In einer weiteren bevorzugten Ausführungsform der Erfindung werden als Komponente b) Verbindungen bix) und/oder bx) oder Gemische von Verbindungen bix) und/oder bx) verwendet, wobei die durchschnittliche Anzahl von olefinischen, radikalisch polymerisierbaren Doppelbindungen pro Molekül mehr als 2 beträgt. Solche Gemische ergeben sich beispielsweise durch Mischen von Verbindungen mit jeweils 2 und Verbindungen mit jeweils 3 oder mehr polymerisierbaren Doppelbindungen pro Molekül. Natürlich können in den Gemischen auch Verbindungen enthalten sein, die nur eine oder keine Doppelbindung pro Molekül enthalten. Solche Verbindungen sind dann aber in derartigen Mengen enthalten, dass die durchschnittliche Zahl von polymerisierbaren Doppelbindungen pro Molekül trotzdem mehr als 2 beträgt.

Es soll an dieser Stelle betont werden, dass es als Komponente b) geeignete Verbindungen gibt, die beiden Gruppen bix) und bx) zugeordnet werden können, da sie sowohl Ester- als auch Ethergruppen enthalten. Kommerziell erhältliche Produkte, die als Komponente b) geeignet sind, sind beispielsweise:
Photomer^{®}5010, Photomer^{®}5429, Photomer^{®}5430, Photomer^{®}5432, Photomer^{®}5662, Photomer^{®}5806, Photomer^{®}5930 der Firma Cognis;
die Resin^{®}-Marken der Firma UCB wie z.B. Resin^{®}80, 81, 83, 450, 657, 770, 809, 810, 830, 835, 870, 1657, 1810, 1870, 20477**, 2870;
die CN^{®}-Marken der Firma Sartomer wie z.B. CN293, CN294, CN296, CN292, CN2297A, CN2279, CN2280, CN2470, CN295, CN2300, CN2200, CN2203, CN2282, CN2284, CN2270, CN2271, CN2272, CN2273, CN2276, CN2250, CN2251, CN2252, CN2253, CN2255, CN2256, CN2257, CN2258, CN2259, CN2260, CN2261;
AROPLAZ^{®}4097-WG4-55 der Firma Reichhold;
Syntholux^{®}-PE-Marken der Firma Synthopol als Polyesteracrylate und die Syntholux^{®}-PA-Marken der Firma Synthopol als Polyetheracrylate;
Laromer^{®}-Marken Laromer^{®}PE 55F, Laromer^{®}PE 56F, Laromer^{®}PE 46T, Laromer^{®}9004, Laromer^{®}PE 44F, Laromer^{®}8800. Laromer^{®}LR 8981, Laromer^{®}LR 8992, Laromer^{®}PE 22WN, Laromer^{®}PE 55WN, Laromer^{®}PO 33F, Laromer^{®}LR 8863, Laromer^{®}PO 43F, Laromer^{®}LR 8967, Laromer^{®}LR 8982, Laromer^{®}LR 9007 (BASF).

### bxi) Verbindungen mit wenigstens zwei radikalisch polymerisierbaren Doppelbindungen

Als Komponente b) kommen auch Verbindungen mit wenigstens zwei radikalisch polymerisierbaren, olefinisch ungesättigten Doppelbindungen pro Molekül in Betracht. Solche Verbindungen werden üblicherweise als Vernetzer bezeichnet. Radikalisch polymerisierbare, olefinisch ungesättigte Doppelbindungen sind beispiels-weise Alkenylgruppen, die formal durch Ablösen eines H-Atoms aus einem Alken ent-stehen. Dazu gehören Vinyl- (-CH=CH₂), 1-Propenyl (-CH=CH-CH₃), 2-Propenyl oder Allyl (-CH₂-CH=CH₂), 1-Butenyl (-CH=CH-CH₂-CH₃) usw..

Auch Alkylidengruppen, also Gruppen, die mit einem Kohlenstoffatom eines Moleküls durch eine Doppelbindung verknüpft sind, gehören zu den radikalisch polymerisierba-ren, olefinisch ungesättigte Doppelbindungen (Beispiel Ethyliden: =CHCH₃).

Geeignete Vernetzer sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei olefinisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypiva-linsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000.

Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethy-lolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C3- bis C6-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Vernetzer sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylam-moniumchlorid oder -methylsulfat, als Vernetzer geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N,N'-Divinylethylenharnstoff oder N,N'-Divinylpropylenharnstoff.

Geeignet sind auch Alkylenbisacrylamide wie Methylenbisacrylamid und N,N'-(2,2-) butan und 1,1'-bis-(3,3'-vinylbenzimidazolith-2-on)-1,4-butan.

Andere geeignete Vernetzer sind beispielsweise Alkylenglykoldi(meth)acrylate wie Ethylenglykoldiacrylat, Ethylenglykoldimethacrylat, Tetraethlyenglykolacrylat, Tetraethylenglykoldimethacrylat, Diethylenglykolacrylat, Diethylenglykolmethacrylat, Vinylacrylat, Allylacrylat, Allylmethacrylat, Divinyldioxan, Pentaerythritallylether sowie Gemische dieser Vernetzer.

Weitere geeignete Vernetzer sind Divinyldioxan, Tetraallylsilan oder Tetravinylsilan. Besonders bevorzugt eingesetzte Vernetzer sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Allylmethacrylat, Pentaerythrittriallylether, Methylenbisacrylamid, N,N'-Divinylethylenharnstoff, Triallylamin und Triallylmonoalkylammoniumsalze, und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

### bxii) von aii) verschiedene (Meth)acrylamide wie z.B. Acrylamid, Methacrylamid, N-Methyl(meth)acrylamid, N-Ethyl(meth)acrylamid, N-n-Propyl-(meth)acrylamid und N-i-Propyl-(meth)acrylamid.

Bevorzugt als Verbindungen b) sind anionische oder anionogene Verbindungen bi), Aminogruppen tragende Verbindungen bii) oder biv), wasserlösliche, nichtionische Verbindungen bvi), Urethangruppen tragende Verbindungen bvii), Polyester bix), Polyether bx) und Mischungen daraus.

### Lösungspolymerisation

Erfindungsgemäß werden die Polymere durch Lösungspolymerisation in einem Alkohol umfassenden Lösungsmittel hergestellt, das im Bereich von 5 bis 50 Gew.-% Wasser enthält.

Geeignete Alkohole sind beispielsweise Methanol, Ethanol, n-Propanol, Isopropanol, n-Butanol, sek.-Butanol, tert.-Butanol, 3-Methyl-1-butanol (Isoamylalkohol), n-Hexanol, Cyclohexanol oder Glykole wie Ethylenglykol, Propylenglykol und Butylenglykol sowie Alkylether mehrwertiger Alkohole wie Diethylenglykol, Triethylenglykol, Polyethylenglykolen mit zahlenmittleren Molekulargewichten bis etwa 3000, Glycerin und Dioxan. Besonders bevorzugt umfasst das Lösungsmittel Ethanol und/oder Isopropanol, insbesondere Ethanol.

Zusätzlich zu Alkohol und Wasser können weitere Lösungsmittel eingesetzt werden. Prinzipiell kommen alle für die radikalische Polymerisation in Frage wie beispielsweise Aceton, Acetonitril, Anilin, Anisol, Benzonitril, tert-Butylmethylether (TBME), Gamma-Butyrolacton, Chinolin, Chloroform, Cyclohexan, Diethylether, Dimethylacetamid, Dimethylformamid, Dimethylsulfoxid, Dioxan, Eisessig, Essigsäureanhydrid, Essigsäureethylester, Ethylendichlorid, Ethylenglycoldimethylether, Formamid, Hexan, Methylenchlorid, Methylethylketon, N-Methylformamid, Petrolether/Leichtbenzin, Piperidin, Propylencarbonat (4-Methyl-1,3-dioxol-2-on), Sulfolan, Tetrachlorethen, Tetrachlorkohlenstoff, Tetrahydrofuran, Toluol, 1,1,1-Trichlorethan, Trichlorethen, Triethylenglycoldimethylether (Triglyme).

Bevorzugt enthält das Lösungsmittel im Bereich von 8 bis 45 Gew.-%, besonders bevorzugt 15 bis 45 Gew.- Wasser.

Bevorzugt enthält das Lösungsmittel im Bereich von 55 bis 92 Gew.-%, besonders bevorzugt 55 bis 85 Gew.-% Ethanol und/oder Isopropanol.

Bevorzugt ist ein erfindungsgemäßes Verfahren bei dem die Temperatur, bei der die Polymerisation durchgeführt wird, im Bereich von 30 bis 120°C, bevorzugt von 40 bis 100°C liegt.

Wenn die zur Polymerisation eingesetzt Menge von Monomer a) wenigstens 70 Gew.-% der Menge der insgesamt eingesetzten Monomere beträgt, ist es vorteilhaft, wenn das Gewichtsverhältnis von Wasser zu Alkohol größer als 1:2 ist.

Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 10 bar.

Wenn die zu polymerisierende Monomerzusammensetzung mehr basische als saure Gruppen enthält, so ist es vorteilhaft, den pH-Wert der Polymerisationslösung durch Zugabe von Säuren herabzusetzen.

Werden als Verbindungen b) Vinyllactame bvi) eingesetzt, so wird der pH-Wert vor und während der Polymerisation auf einem Wert von wenigstens 6, bevorzugt im Bereich von 6 bis 8 gehalten.

Mindestens einer der für das erfindungsgemäße Verfahren verwendeten Initiatoren ist ein ethanollöslicher Initiator.

Ethanollöslich bedeutet, dass bei einem Druck von 1bar und einer Temperatur von 20°C und 1013 mbar wenigstens 1g, bevorzugt wenigstens 5g und insbesondere wenigstens 10g des jeweiligen Initiators in 1 Liter Ethanol klar gelöst werden können. Bevorzugt ist der Initiator ausgewählt aus ethanollöslichen Diazo- und Peroxidverbindungen.

Beispiele für solche Initiatoren sind Diacetylperoxid, Dibenzoylperoxid, Succinylperoxid, Di-tert.-butylperoxid, tert.-Butylperbenzoat, tert.-Butylperpivalat, tert.-Butylperoctoat, tert.-Butylperoxy-2-ethylhexanoat, tert.-Butylpermaleinat, Benzoylperoxid, tert.-Amylperoxipivalat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azobisisobutyronitril, 2,2'-Azobis(2-methylpropionamid)dihydrochlorid (Wako^{®}V50), Dimethyl 2,2'-azobis(2-methylpropionat) oder 2,2'-Azobis(2-methyl-butyronitril). Bevorzugt ist der Polymerisationsinitiator ausgewählt aus der Gruppe bestehend aus Benzoylperoxid, tert.-Amylperoxipivalat, 2,2'-Azobis(2-methylpropionamid)-dihydrochlorid, Dimethyl 2,2'-azobis(2-methylpropionat), 2,2'-Azobis(2-methylbutyro-nitril), 2,2'-Azobis(2-methylpropionamid).

Geeignete Initiatoren sind weiterhin 2,2'-Azobis(4-methoxy-2,4-dimethylvaleronitril), 2,2'-Azobis(2,4-dimethyl valeronitril), Dimethyl 2,2'-azobis(2-methylpropionat), 2,2'-Azobis(2-methylbutyronitril), 1,1'-Azobis(cyclohexan-1-carbonitril), 2,2'-Azobis[N-(2-propenyl)-2-methylpropionamid], 1-[(cyano-1-methylethyl)azo] formamid, 2,2'-Azobis(N-butyl-2-methylpropionamid), 2,2'-Azobis(N-cyclohexyl-2-methylpropionamid). Die vorgenannten Azo-Initiatoren sind unter den Wako^{®}V-Marken kommerziell erhältlich.

Die Menge des wenigstens einen für die Polymerisation der Monomeren eingesetzten ethanollöslichen Initiators beträgt vorzugsweise von 0,001 bis 2,5, besonders bevorzugt 0,01 bis 1,5 und insbesondere 0,05 bis 1,0 Gew.-%, bezogen auf die Gesamtmenge der eingesetzten Monomere.

In einer Ausführungsform der Erfindung werden im erfindungsgemäßen Verfahren sowohl wenigstens ein ethanollöslicher als auch wenigstens ein wasserlöslicher Initiator eingesetzt.

Unter einem wasserlöslichen Polymerisationsinitiator wird ein Initiator verstanden, der bei 20°C und 1013 mbar zu wenigstens 1g, bevorzugt zu wenigstens 5g und insbesondere zu wenigstens 10g in 1 Liter Wasser klar löslich ist.

Wasserlösliche Polymerisationsinitiatoren können ausgewählt werden aus der Gruppe bestehend aus Peroxiden, Hydroperoxiden, Peroxodisulfaten, Percarbonaten, Peroxidestern, Azoverbindungen und deren Mischungen.

In einer Ausführungsform der Erfindung wird der zusätzlich zum ethanollöslichen Initiator verwendete wasserlösliche Polymerisationsinitiator bevorzugt ausgewählt aus der Gruppe bestehend aus wasserlöslichen Azoverbindungen, Wasserstoffperoxid, Lithiumperoxodisulfat, Natriumperoxodisulfat, Kaliumperoxodisulfat, Ammoniumperoxodisulfat, und deren Mischungen.

Weiterhin bevorzugt wird der zusätzlich zum ethanollöslichen Initiator verwendete wasserlösliche Polymerisationsinitiator ausgewählt aus der Gruppe bestehend aus der Gruppe bestehend aus 2,2'-Azobis[2-(5-methyl-2-imidazolin-2-yl)propan]dihydrochlorid 2,2'-Azobis[2-(2-imidazolin-2-yl)propan] dihydrochlorid 2,2'-Azobis[2-(2-imidazolin-2-yl)propandisulfatdihydrat 2,2'-Azobis(2-methylpropionamid)dihydrochlorid 2,2'-Azobis[N-(2-carboxyethyl)-2-methylpropionamidin]tetrahydrat 2,2'-Azobis[2-(3,4,5,6-tetrahydropyrimidin- 2-yl)propan] dihydrochlorid 2,2'-Azobis{2-[1-(2-hydroxyethyl)-2-imidazolin-2-yl]propan}dihydrochlorid 2,2'-Azobis[2-(2-imidazolin-2-yl)propan] 2,2'-Azobis{2-methyl-N-[1,1-bis(hydroxymethyl)-2-hydroxyethyl]propionamid 2,2'-Azobis{2-methyl-N-[2-(1-hydroxybuthyl)]propionamid} 2,2'-Azobis[2-methyl-N-(2-hydroxyethyl)propionamid] und deren Mischungen.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

Die Mengen an zu polymerisierenden Verbindungen, bezogen auf Lösungsmittel, werden vorzugsweise so gewählt, dass Lösungen mit einem Feststoffgehalt von 25 bis 80 Gew.-% erhalten werden.

Die Lösungspolymerisation kann sowohl als Batchprozess als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzentrationsgefälles unter Aufrechterhaltung der Polymerisation der Polymerisationszone zuführt.

Zur Erzielung möglichst reiner Polymere mit geringem Restmonomergehalt schlieβt sich an die Hauptpolymerisation eine Nachpolymerisation an. Die Nachpolymerisation erfolgt in Gegenwart eines wasserlöslichen Initiators.

Die Hauptpolymerisation gilt als beendet, wenn der Restmonomergehalt höchstens 10, bevorzugt höchstens 5, besonders bevorzugt höchstens 2 und insbesondere höchstens 1 Gew.-%, bezogen auf den Feststoffgehalt der Polymerisationsmischung, beträgt.

Eine bevorzugte Ausführungsform der Erfindung ist also das erfindungsgemäße Verfahren, dadurch gekennzeichnet, dass die Polymerisation bis zum Erreichen eines Restmonomergehalts von höchstens 5, besonders bevorzugt höchstens 2 und insbesondere höchstens 1 Gew.-% (Hauptpolymerisation), bezogen auf den Feststoffgehalt der Polymerisationsmischung, in Gegenwart von wenigstens einem ethanollöslichen Initiator und die sich an die Hauptpolymerisation anschließende Nachpolymerisation in Gegenwart von wenigstens einem wasserlöslichen Initiator durchgeführt wird.

Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden.

Die zur Polymerisation eingesetzten Monomeren werden vorzugsweise zu wenigstens 95, besonders bevorzugt zu wenigstens 99 und insbesondere zu wenigstens 99,9% umgesetzt (Polymerisationsgrad).

Die nach der Polymerisation in Lösung vorliegenden Polymere können durch übliche, dem Fachmann bekannte Trocknungsverfahren in Pulver überführt werden. Bevorzugte Verfahren sind beispielsweise die Sprühtrocknung, die Sprühwirbelschichttrocknung, die Walzentrocknung und die Bandtrocknung. Ebenfalls anwendbar sind die Gefriertrocknung und die Gefrierkonzentrierung. Gewünschtenfalls kann das Lösungsmittel auch durch übliche Methoden, z. B. Destillation bei verringertem Druck, teilweise oder vollständig entfernt werden.

Eine bevorzugte Ausführungsform der Erfindung ist das erfindungsgemäße Verfahren, dadurch gekennzeichnet, dass das als Lösungsmittel-Bestandteil verwendete Isopropanol nach der Polymerisation im wesentlichen vollständig entfernt wird, wobei die Entfernung vorzugsweise durch Destillation erfolgt.

Wenn zur erfindungsgemäßen Herstellung des Polymeren n-Vinylpyrrolidon als Monomer b) und Ethanol als Lösungsmittelbestandteil eingesetzt werden, so ist es vorteilhaft, wenn das Ethanol nach der Polymerisation im wesentlichen vollständig entfernt wird.

Unter "im wesentlichen" entfernen eines Bestandteils wird eine Entfernung des ursprünglich im Lösungsmittel vorhandenen Bestandteils bis zu einem Restgehalt von höchstens 10, bevorzugt höchstens 5 und insbesondere höchstens 1 Gew.-% dieses Bestandteils, bezogen auf die gesamte Polymerisationsmischung, verstanden.

Wenn eine Destillation zur Entfernung des alkoholischen Lösungsmittels durchgeführt werden soll, so ist es von Vorteil, den pH-Wert der Polymerisationslösung nach der Polymerisation auf einen Wert von 6,5 oder weniger, vorzugsweise auf einen Wert zwischen 4 und 6,5 einzustellen.

Der Feststoffgehalt der Polymerisationslösung, also die Menge aller enthaltenen Komponenten mit Ausnahme der Lösungsmittel beträgt bevorzugt wenigstens 20, besonders bevorzugt wenigstens 25 und insbesondere wenigstens 30 Gew.-%.

Bei den durch das erfindungsgemäße Verfahren hergestellten Polymeren kann es sich um anionische bzw. anionogene Polymere handeln. Für die Verwendung in kosmetischen Zubereitugnen ist es vorteilhaft, wenn die Säuregruppen dieser Polymere mit einer Base teilweise oder vollständig neutralisiert werden, da die erhaltenen Salze der Polymere in der Regel eine bessere Löslichkeit oder Dispergierbarkeit in Wasser aufweisen als die nicht neutralisierten Polymere. Als Base für die Neutralisation dieser Polymere können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethyanolamine, bevorzugt Methyl- oder Ethyldiethanolamin und Di-C₁-C₆-Alkylethanolamine. Besonders für den Einsatz in Haarbehandlungsmitteln haben sich zur Neutralisation von Säuregruppen enthaltenden Polymeren 2-Amino-2-methyl-1-propanol, 2-Amino-2-ethylpropan-1,3-diol, Diethylaminopropylamin, Triethanolamin und Triisopropanolamin bewährt. Das Neutralisationsmittel kann beispielsweise auch 2-Amino-2-methyl-1-propanol oder eine Mischung aus 2-Amino-2-methyl-1-propanol und Triethanolamin sein oder umfassen.

Die Neutralisation der Säuregruppen enthaltenden Polymere kann auch mit Hilfe von Mischungen mehrerer Basen vorgenommen werden, z. B. Mischungen aus Natronlauge oder Kalilauge.

Die Neutralisation kann je nach Anwendungszweck partiell z. B. zu 5 bis 95 %, vorzugsweise 30 bis 95 %, oder vollständig, d. h. zu 100 % erfolgen. Weiterhin kann das Neutralisationsmittel auch in mehr als äquivalenter Menge zugesetzt werden.

### Kosmetische Zubereitungen

Strengere Umweltauflagen und wachsendes ökologisches Bewusstsein fordern zunehmend immer geringere Anteile an flüchtigen organischen Komponenten (englisch: volatile organic compounds, VOC) in kosmetischen Aerosol-Zubereitungen wie beispielsweise Aerosol-Haarsprays.

Der VOC-Gehalt in Haarsprays wird im wesentlichen durch die nicht-wässrigen Lösungsmittel und die Treibmittel bestimmt. Daher wird gegenwärtig verstärkt anstelle von nicht-wässrigen Lösungsmittel auf Wasser als Lösungsmittel zurückgegriffen. Dieser Ersatz der organischen Lösungsmitteln bringt allerdings einige Probleme mit sich. So sind Formulierungen der aus dem Stand der Technik bekannten filmbildenden Polymere, die entsprechende VOC-Auflagen erfüllen, beispielsweise nicht oder erst nach weiterer Verdünnung sprühbar und somit nur bedingt für die Verwendung in Haarsprays geeignet. Polymerfilme, die aus solchen Zubereitungen entstehen, weisen mitunter nicht die notwendige mechanische Qualität und somit ungenügende Festigungswirkung und schlechten Halt für das Haar auf.

Die durch das erfindungsgemäße Verfahren hergestellten Copolymere eignen sich hervorragend zur Herstellung kosmetischer, insbesondere haut- und/oder haarkosmetischer Zubereitungen. Sie dienen dabei z.B. als polymere Filmbildner. Sie sind universell in den verschiedensten kosmetischen, bevorzugt haarkosmetischen Zubereitungen einsetzbar und einformulierbar und mit den üblichen Zusatz-Komponenten verträglich.

Die Copolymere eignen sich vorteilhaft zur Erzeugung elastischer Frisuren bei gleichzeitig starker Festigung, auch bei hoher Luftfeuchtigkeit. Die Copolymere zeichnen sich in Aerosolformulierungen durch gute Treibgasverträglichkeit, gute Löslichkeit in wässrig/alkoholischen Lösungsmittelgemischen, insbesondere durch die Eignung für einen Einsatz als optisch klare Low-VOC-Formulierungen und durch gute Auswaschbarkeit und Auskämmbarkeit ohne Flaking-Effekt aus. Zudem verbessern sie mit ihnen behandeltes Haar in seinen sensorisch erfassbaren Eigenschaften wie Griff, Volumen oder Handhabbarkeit. Haarsprayformulierungen auf Basis der durch das erfindungsgemäße Verfahren hergestellten Copolymere zeichnen sich durch gute Sprühbarkeit und gute rheologische Eigenschaften und äußerst geringe Klebrigkeit der resultierenden Filme aus. Die die Copolymere enthaltenden kosmetischen, bevorzugt haarkosmetischen Zubereitungen neigen nach dem Aufbringen nicht zur Schaumbildung. Neben der guten Verträglichkeit mit den üblichen kosmetischen Inhaltsstoffen trocknen die aufgetragenen Copolymer-Filme schnell.

Ein weiterer Gegenstand der vorliegenden Erfindung ist demnach die Verwendung der durch das erfindungsgemäße Verfahren erhältlichen Copolymere in kosmetischen Zubereitungen sowie solche kosmetischen Zubereitungen an sich.

### Kosmetisch akzeptabler Träger B

Bevorzugt handelt es sich bei den kosmetischen Zubereitungen um wässrige Zubereitungen, die wenigstens 10, bevorzugt wenigstens 20 und besonders bevorzugt wenigstens 30 Gew.-% Wasser enthalten. Bevorzugt enthalten die erfindungsgemäßen kosmetischen Zubereitungen höchstens 80 (VOC-80), bevorzugt höchstens 55 Gew.-% (VOC-55) flüchtige organische Bestandteile.

Ein Gegenstand der Erfindung sind demnach kosmetische Zubereitungen, bei denen der Anteil an flüchtigen organischen Komponenten höchstens 55 Gew.%, bezogen auf die kosmetische Zubereitung, beträgt.

Die kosmetischen Zubereitungen weisen neben Wasser und den durch das erfindungsgemäße Verfahren erhältlichen Copolymeren weiterhin wenigstens einen kosmetisch akzeptablen Träger B auf, der ausgewählt ist unter
i) wassermischbaren organischen Lösungsmitteln, vorzugsweise C₂-C₄-Alkanolen, insbesondere Ethanol,
ii) Ölen, Fetten, Wachsen,
iii) von ii) verschiedenen Estern von C₆-C₃₀-Monocarbonsäuren mit ein-, zwei- oder dreiwertigen Alkoholen,
iv) gesättigten acyclischen und cyclischen Kohlenwasserstoffen,
v) Fettsäuren,
vi) Fettalkoholen,
vii) Treibmitteln (Treibgasen) und
viii) Mischungen davon.

Geeignete Träger B und weitere, vorteilhaft zu verwendende Wirk- und Zusatzstoffe sind im Folgenden detailliert beschrieben.

Geeignete kosmetisch und pharmazeutisch verträgliche Öl- bzw. Fettkomponenten sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Die kosmetischen Zubereitungen können z.B. als kosmetisch akzeptablen Träger B eine Öl- bzw. Fettkomponente aufweisen, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkemöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc..

Bei den kosmetischen Zubereitungen kann es sich um hautkosmetische, haarkosmetische oder dermatologische, hygienische oder pharmazeutische Zubereitungen handeln. Aufgrund ihrer filmbildenden und flexiblen Eigenschaften eignen sich die durch das erfindungsgemäße Verfahren herstellbaren Copolymere insbesondere als Zusatzstoffe für Haar- und Hautkosmetika.

Vorzugsweise liegen die kosmetischen Zubereitungen, die die erfindungsgemäßen Copolymere enthalten, in Form eines Sprays, Gels, Schaums, einer Salbe, Creme, Emulsion, Suspension, Lotion, Milch oder Paste vor. Gewünschtenfalls können auch Liposomen oder Mikrosphären eingesetzt werden.

Vorzugsweise enthalten die erfindungsgemäßen kosmetischen Mittel wenigstens ein erfindungsgemäßes Copolymer, wenigstens einen wie vorstehend definierten Träger B und wenigstens einen davon verschiedenen Bestandteil, der vorzugsweise ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmittein, Gelbildnern, Pflegemitteln, Färbemitteln, Tönungsmitteln, Bräunungsmitteln, Farbstoffen, Pigmenten, Konsistenzgebem, Feuchthaltemitteln, Rückfettern, Collagen, Eiweisshydrolysaten, Lipiden, Antioxidantien, Entschäumern, Antistatika, Emollienzien und Weichmachern.

Die erfindungsgemäßen Zubereitungen besitzen bevorzugt einen pH-Wert von 2,0 bis 9,3. Besonders bevorzugt ist der pH-Bereich zwischen 4 und 8. Als zusätzliche Co-Solventien können organische Lösungsmittel oder ein Gemisch aus Lösungsmitteln mit einem Siedepunkt unter 400°C in einer Menge von 0,1 bis 15 Gew.%, bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.%.

In einer bevorzugten Ausführungsform der Erfindung weisen die kosmetischen Zubereitungen einen Anteil an flüchtigen organischen Komponenten von höchstens 80 Gew.-%, bevorzugt höchstens 55 Gew.-% und insbesondere höchstens 35 Gew.-% auf. Ein bevorzugter Gegenstand sind somit kosmetische, bevorzugt haarkosmetische Zubereitungen, die dem low-VOC-Standard, also VOC-80- bzw. VOC-55-Standard entsprechen.

Bevorzugt ist die Verwendung der Copolymere insbesondere in Haarsprayzubereitungen, welche die folgenden Bestandteile enthalten:
- teilweise oder vollständig neutralisiertes erfindungsgemäßes Copolymer;
- Wasser;
- kosmetisch übliches organisches Lösungsmittel wie beispielsweise Ethanol, Isopropanol und Dimethoxymethan, daneben auch Aceton, n-Propanol, n-Butanol, 2-Methoxypropan-1-ol, n-Pentan, n-Hexan, Cyclohexan, n-Heptan, n-Octan oder Dichlormethan oder deren Gemische;
- kosmetisch übliches Treibmittel wie beispielsweise n-Propan, iso-Propan, n-Butan, i-Butan, 2,2-Dimethylbutan, n-Pentan, Isopentan, Dimethylether, Difluorethan, Fluortrichlormethan, Dichlordifluormethan oder Dichlortetrafluorethan, HFC-152 A (1,1-Difluorethan), HFC-134a (1,1,2,2-Tetrafluorethan), N₂, N₂O und CO oder deren Gemische.

Zur Neutralisation der durch das erfindungsgemäße Verfahren erhältlichen Copolymere und zur Einstellung des pH-Werts der kosmetischen, bevorzugt haarkosmetischen Zubereitungen werden vorteilhaft Alkanolamine eingesetzt. Beispiele sind Aminomethylpropanol, Diethanolamin, Diisopropanolamin, Ethanolamin, Methylethanolamin, N-Lauryl-Diethanolamin, Triethanolamin und Triisoproanolamin. Es können sowohl primäre Aminogruppen tragende als auch sekundäre Aminogruppen tragende Alkanolamine verwendet werden.

Außerdem können Alkalihydroxide (z.B. NaOH, bevorzugt KOH) und andere Basen zur Neutralisation verwendet werden (z.B. Histidin, Arginin, Lysin oder Ethylenediamine, Diethylentriamin, Melamin, Benzoguanamin). Alle angegebenen Basen können allein oder als Gemisch mit anderen Basen zur Neutralisation säurehaltiger kosmetischer Produkte eingesetzt werden.

In einer bevorzugten Ausführungsform der Erfindung werden zur Neutralisation Hydroxygruppen enthaltende Amine aus der Gruppe bestehend aus N,N-Dimethylethanolamin, N-Methyldiethanolamin, Triethanolamin, 2-Amino-2-Methyl-propanol und Mischungen daraus ausgewählt.

Dabei können sekundäre oder tertiäre Aminogruppen tragende Alkanolamine vorteilhafte Wirkungen zeigen.

Ein Gegenstand der vorliegenden Erfindung sind demnach wässrige kosmetische, bevorzugt haut- und/oder haarkosmetische Zubereitungen die neben dem wenigstens einen nach dem erfindungsgemäßen Verfahren erhältlichen Copolymer und dem Träger B mindestens noch einen Wirk- oder Zusatzstoff ausgewählt aus der Gruppe bestehend aus viskositätsmodifizierenden Stoffen, haarpflegenden Stoffen, haarfestigenden Stoffen, Silikonverbindungen, Lichtschutzstoffen, Fetten, Ölen, Wachsen, Konservierungsmitteln, Pigmenten, löslichen Farbstoffen, partikelförmigen Stoffen und Tensiden enthalten.

Derartige haarkosmetische Formulierungen enthalten in einer bevorzugten Ausführungsform
i) 0,05 bis 20 Gew.-% wenigstens eines wie zuvor beschriebenen Copolymers,
ii) 20 bis 99,95 Gew.-% Wasser und/oder Alkohol,
iii) 0 bis 50 Gew.-% wenigstens eines Treibgases,
iv) 0 bis 5 Gew.-% wenigstens eines Emulgators,
v) 0 bis 3 Gew.-% wenigstens eines Verdickers, sowie
vi) bis zu 25 Gew.-% weitere Bestandteile.

Unter Alkohol sind alle vorgenannten, in der Kosmetik üblichen Alkohole zu verstehen, vorzugsweise Ethanol, Isopropanol, n-Propanol.

### Treibmittel (Treibgase)

Als Treibmittel (Treibgase) kommen von den genannten Verbindungen vor allem die Kohlenwasserstoffe, insbesondere Propan, n-Butan, n-Pentan und Gemische hieraus sowie Dimethylether und Difluorethan zur Anwendung. Gegebenenfalls werden einer oder mehrere der genannten chlorierten Kohlenwasserstoffe in Treibmittelmischungen mitverwendet, jedoch nur in geringen Mengen, etwa bis zu 20 Gew.-%, bezogen auf die Treibmittelmischung.

Die kosmetischen Zubereitungen eignen sich auch besonders für Pumpsprayzubereitungen ohne den Zusatz von Treibmitteln oder auch für Aerosolsprays mit üblichen Druckgasen wie Stickstoff, Druckluft oder Kohlendioxid als Treibmittel.

Eine wasserhaltige Standard-Aerosol-Sprayformulierung umfasst beispielsweise folgende Bestandteile:
■ zu 100 % neutralisiertes Copolymer
■ Alkohol
■ Wasser
■ Dimethylether und/oder Propan/ n-Butan und/oder Propan/iso-Butan,

Dabei beträgt die Gesamtmenge der flüchtigen organischen Komponenten bevorzugt höchstens 80, besonders bevorzugt höchstens 55 Gew.-% der Zubereitung.

Vorzugsweise enthalten die kosmetischen Zubereitungen wenigstens ein wie zuvor beschriebenes Copolymer, wenigstens einen wie vorstehend definierten kosmetisch akzeptablen Träger B und wenigstens einen weiteren, davon verschiedenen Wirk- oder Zusatzstoff, der ausgewählt ist unter kosmetisch aktiven Wirkstoffen, Emulgatoren, Tensiden, Konservierungsmitteln, Parfümölen, Verdickern, Haarpolymeren, Haarconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollentien, Lanolin-Komponenten, Proteinhydrolysaten und Weichmachern.

### Weitere Polymere

Zur gezielten Einstellung der Eigenschaften kosmetischer Zubereitungen kann es von Vorteil sein, die zuvor beschriebenen Copolymere in Mischung mit weiteren (haar)kosmetisch üblichen Polymeren einzusetzen.

In einer weiteren bevorzugten Ausführungsform enthält die kosmetische Zubereitung 0,01 bis 15 Gew.%, vorzugsweise 0,5 bis 10 Gew.% mindestens eines weiteren synthetischen oder natürlichen nichtionischen, bevorzugt eines filmbildenden Polymers. Unter natürlichen Polymeren werden auch chemisch modifizierte Polymere natürlichen Ursprungs verstanden. Unter filmbildenden Polymeren werden solche Polymere verstanden, die bei Anwendung in 0,01 bis 5%iger wässriger, alkoholischer oder wässrigalkoholischer Lösung in der Lage sind, auf dem Haar einen Polymerfilm abzuscheiden.

Als solche weiteren üblichen Polymere eignen sich dazu beispielsweise anionische, kationische, amphotere, zwitterionische und neutrale Polymere. Solche Polymere sind dem Fachmann bekannt und bedürfen keiner weiteren Erklärung.

### Beispiele

Die folgenden Beispiele sollen die Erfindung erläutern, ohne sie darauf zu beschränken

### Verwendete Abkürzungen:

- t-BA: tert.-Butylacrylat
- MAS: Methacrylsäure
- AS: Acrylsäure
- NtBAEMA: N-tert.-Butylaminoethylmethacrylat
- VP: N-Vinylpyrrolidon
- MMA: Methylmethacrylat
- DMAEMA: Dimethylaminoethylmethacrylat
- t-BMA: tert.-Butylmethacrylat
- i-BMA: Isobutylmethacrylat
- EMA: Ethylmethacrylat
- SMA: Stearylmethacrylat
- UDA: Urethandiacrylat¹⁾
- UA3: Urethanacrylat 3
- Si-UA: DE 198 38 852, Tab.1, Bsp. Nr. 2
- iPrOH: Iso-Propanol
- EtOH: Ethanol
- VE: voll entsalzt
- V59: Initiator Wako^{®}V59
- TBPP: Tert.-Butylperpivalat
- TBPO: Tert.-Butylperoctoat
- NaPS: Natriumperoxodisulfat
- AMP: 2-Amino-2-Methylpropanol

### Herstellung von Urethanacrylat 3

In einem Rundkolben wurden 672,0g eines Polyesters aus Adipinsäure und Neopentylglykol mit einer OH Zahl von ca. 200, 140,0g Hydroxyethylacrylat, 0,6g Hydrochinonmonomethylether, 1,21 g 2,6, Di-tert. Butyl-4-methylphenol vorgelegt und auf 50°C aufgeheizt. Dann wurden 400,0g Isophorondiisocyanat innerhalb 30 Minuten zugetropft. Man ließ weitere 20 Stunden bei 90-95°C reagieren, wobei der NCO-Gehalt auf 0,1% abfiel. Es wurde auf 60°C abgekühlt, dann wurden 10,0 g Methanol zugegeben und ca. 4 Stunden bei 90-95°C weiterreagieren lassen bis der Isocyanatgehalt (NCO-Wert) auf 0 abgefallen war. Das erhaltene Harz wurde bei Raumtemperatur mit 510,0g Tripropylenglykoldiacrylat gemischt und über einen 50 µm Filter filtriert und abgefüllt.

### Herstellung von Urethanacrylat A

In einem Rundkolben wurden 672,0g eines Polyesters aus Adipinsäure und Neopentylglykol mit einer OH-Zahl von ca. 200, 140,0g Hydroxyethylacrylat, 0,6 g Hydrochinon-monomethylether, 1,20g 2,6, Di-tert. Butyl-4-methylphenol, 0,12 g Tetrabutylortho-titanat vorgelegt und auf 50°C aufgeheizt. Dann wurden 400, 0 g Isophorondiisocyanat innerhalb 30 Minuten zugetropft. Man ließ weitere 7 Stunden bei 90-95°C reagieren, wobei der NCO-Gehalt auf 0,56% abfiel. Es wurde auf 60°C abgekühlt, dann wurden 520,0 g Ethanol zugegeben und ca. 2 Stunden bei 65-70°C weiterreagieren lassen bis der Isocyanatgehalt (NCO-Wert) auf 0 abgefallen war. Das erhaltene Harz wurde über einen 50 µm Filter filtriert und abgefüllt.

### Herstellung von Copolymer gemäß Beispiel 1:

Polymer aus t-BA, MAS und AS im Gewichtsverhältnis 75: 20: 5

| | | |
|---|---|---|
| Vorlage | 100 g | VE-Wasser |
| | 200 g | Ethanol |
| | 35 g | Zulauf 1 |
| | 5 g | Zulauf 2 |
| | | |
| Zulauf 1 | 300 g | tert.-Butylacrylat |
| | 80 g | MAS |
| | 20 g | AS |
| | 225 g | Ethanol |
| | | |
| Zulauf 2 | 120 g | Ethanol |
| | 6 g | Wako^{®}V59 (Herst.: Wako Pure Chemical Industries Ltd.) |
| | | |
| Zulauf 3 | 180 g | Ethanol |
| | 8 g | tert.- Butylperpivalat |
| | | |
| Zulauf 4 | 118 g | AMP (90%ig) |
| | 112 g | Wasser |

Vorlage wurde unter Stockstoff-Atmosphäre auf 75°C erwärmt. Die Zuläufe 1 und 2 wurden innerhalb von 3 Stunden zugegeben. Die Polymerlösung wurde für weitere 2 Stunden bei 78°C weitergerührt. Zulauf 3 wurde in 30 min. zudosiert und anschließend wurde noch weitere 5 h bei 80°C gerührt. Schließlich wurde Zulauf 4 zugegeben und während 30 min. neutralisiert.

Ungefähr 10 Gew.-% des eingesetzten Ethanols wurden bei einer Badtemperatur von 120°C und einer Übergangstemperatur von etwa 80°C abdestilliert. Der Lösung wurde anschließend die durch Destillation entfernte Menge Ethanol in Form von frischem Ethanol wieder zugegeben.

Analog wurden die Polymere gemäß der Beispiele 2, 3 und 9 hergestellt.

### Herstellung von Copolymer gemäß Beispiel 5:

Polymer aus t-BA, MAS, VP, DMAEMA im Gewichtsverhältnis 52: 3: 27:18

| | | |
|---|---|---|
| Vorlage | 200 g | VE-Wasser |
| | 265 g | Ethanol |
| | 35 g | Zulauf 1 |
| | 5 g | Zulauf 2 |
| | | |
| Zulauf 1 | 156 g | t-BA |
| | 108 g | VP |
| | 72 g | DMAEMA |
| | 12 g | MAS |
| | 200 g | Ethanol |
| | | |
| Zulauf 2 | 80 g | Ethanol |
| | 4 g | Wako^{®}V59 |
| | | |
| Zulauf 3 | 120 g | Ethanol |
| | 6 g | tert.- Butylperpivalat |
| | | |
| Zulauf 4 | 115 g | Milchsäure (90%ig) |
| | 67 g | Wasser |
| | | |
| Zulauf 5 | 400 g | Ethanol |

Die Vorlage wurde unter Stickstoff-Atmosphäre auf 75°C erwärmt. Zulauf 1 wurde innerhalb von in 4 Stunden und Zulauf 2 innerhalb von 5 Stunden zugegeben. Die Polymerlösung wurde dann bei 75°C noch 2 weitere Stunden gerührt. Zulauf 3 wurde dann in 30 min. zudosiert und die Lösung anschließen noch weitere 4 Stunden bei 80°C gerührt. Schließlich wurde durch Zugabe von Zulauf 4 während 20 min. neutralisiert.

Bei einer Badtemperatur von 120°C wurde so lange Ethanol abdestilliert bis eine Innentemperatur von 85°C erreicht war. Anschließend wurde mit Wasserdampf bis zum Erreichen einer Innentemperatur von ca.100°C destilliert. Schließlich wurde auf ca. 40°C gekühlt, dann Zulauf 5 zudosiert und bis zum Vorliegen einer homogenen Phase gerührt. Wasser wurde bis zum Erreichen eines Feststoffgehaltes von 30 Gew.-% zugegeben.

Analog dazu wurden die Polymere gemäß der Beispiele 6 und 7 hergestellt.

### 3.) Herstellung von Copolymer gemäß Beispiel 4:

Polymer aus MMA, MAS, AS und DMAEMA im Gew.-Verhältnis 73: 12: 12: 3

| | | |
|---|---|---|
| Vorlage | 215 g | VE-Wasser |
| | 200 g | iso-Propanol |
| | 35 g | Zulauf 1 |
| | 5 g | Zulauf 2 |
| | | |
| Zulauf 1 | 292 g | Methylmethacrylat |
| | 48 g | MAS |
| | 48 g | AS |
| | 12 g | DMAEMA |
| | 225 g | iso-Propanol |
| | | |
| Zulauf 2 | 75 g | iso-Propanol |
| | 7,8 g | Wako^{®}V59 |
| | | |
| Zulauf 3 | 230 g | iso-Propanol |
| | 4 g | tert.- Butylperpivalat |
| | | |
| Zulauf 4 | 55 g | AMP |
| | 55 g | Wasser |
| | | |
| Zulauf 5 | 400 g | Ethanol |

Die Vorlage wurde unter Stickstoff-Atmosphäre auf 75°C erwärmt. Zulauf 1 wurde innerhalb von in 4 Stunden und Zulauf 2 innerhalb von 5 Stunden zugegeben. Die Polymerlösung wurde dann bei 75°C noch 2 weitere Stunden gerührt. Zulauf 3 wurde dann in 30 min. zudosiert und die Lösung anschließen noch weitere 4 Stunden bei 80°C gerührt. Schließlich wurde durch Zugabe von Zulauf 4 während 30 min. neutralisiert.

Bei einer Badtemperatur von 120°C wurde so lange iso-Propanol abdestilliert bis eine Innentemperatur von 85°C erreicht war. Anschließen wurde mit Wasserdampf bis zum Erreichen einer Innentemperatur von ca.100°C destilliert.

Schließlich wurde auf ca. 40°C gekühlt, dann Zulauf 5 zudosiert und bis zum Vorliegen einer homogenen Phase gerührt. Wasser wurde bis zum Erreichen eines Feststoffgehaltes von 30 Gew.-% zugegeben.

Analog dazu wurden die Polymere gemäß der Beispiele 8 und 10 hergestellt.

### Herstellung von Copolymer gemäß Beispiel 11:

Polymer aus MMA, MAS, AS und Urethandiacrylat (Laromer®UA 19T) im Gew.-Verhältnis 74: 12: 12: 2

| | | |
|---|---|---|
| Vorlage | 327 g | VE-Wasser |
| | 200 g | Ethanol |
| | 35 g | Zulauf 1 |
| | 5 g | Zulauf 2 |
| | | |
| Zulauf 1 | 296 g | Methylmethacrylat |
| | 48 g | MAS |
| | 48 g | AS |
| | 8 g | Laromer^{®}UA 19T |
| | 105 g | Ethanol |
| | | |
| Zulauf 2 | 58 g | Ethanol |
| | 3.9 g | Wako^{®}V59 |
| | | |
| Zulauf 3 | 60 g | Ethanol |
| | 2 g | tert.- Butylperpivalat |
| | | |
| Zulauf 4 | 55 g | AMP |
| | 55 g | Wasser |
| | | |
| Zulauf 5 | 400 g | Ethanol |

Die Vorlage wurde unter Stickstoff-Atmosphäre auf 75°C erwärmt. Zulauf 1 wurde innerhalb von in 4 Stunden und Zulauf 2 innerhalb von 5 Stunden zugegeben. Die Polymerlösung wurde dann bei 78°C noch 2 weitere Stunden gerührt. Zulauf 3 wurde dann in 30 min. zudosiert und die Lösung anschließen noch weitere 4 Stunden bei 80°C gerührt. Schließlich wurde durch Zugabe von Zulauf 4 während 30 min. neutralisiert.

Bei einer Badtemperatur von 120°C wurde so lange Ethanol abdestilliert bis eine Innentemperatur von 85°C erreicht war. Anschließend wurde mit Wasserdampf bis zum Erreichen einer Innentemperatur von ca.100°C destilliert. Schließlich wurde auf ca. 40°C gekühlt, dann Zulauf 5 zudosiert und bis zum Vorliegen einer homogenen Phase gerührt. Wasser wurde bis zum Erreichen eines Feststoffgehaltes von 30 Gew.-% zugegeben.

Analog dazu wurde das Polymer gemäß Beispiel 12 hergestellt.

### Herstellung von Copolymer gemäß Beispiel 13:

| | | |
|---|---|---|
| Zulauf 1 | 657g | Methylmethacrylat |
| | 180g | Methacrylsäure |
| | 45g | Acrylsäure |
| | 18g | Laromer^{®}UA 19 T |
| | | |
| Zulauf 2 | 23g | tert.-Butylperoctoat |
| | 1015g | Ethanol kosm. |
| | 405g | VE-Wasser |

Als Vorlage wurden in einem 5-l-Edelstahlreaktor 45 g von Zulauf 1 mit 450 g kosmetischem Ethanol und 180 g VE-Wasser gemischt. Diese Vorlage wurde mit Stickstoffamosphäre 3mal abgepresst (5,0bar) und dann bei 0,5bar auf 90°C erhitzt. Danach wurden 72.20 g von Zulauf 2 zugegeben. Nach 10 min wurden die Zuläufe 1 und 2 gemeinsam gestartet. Zulauf 1 wurde in 3 Stunden und Zulauf 2 in 4 Stunden bei 90°C unter Eigendruck zudosiert. Die Reaktionsmischung wurde 2 Stunden bei 90°C unter Eigendruck weiter polymerisiert. Danach wurde Zulauf 3 (3.44g tert.-Butylperoctoat, 43.00g Ethanol kosm., 17.00g VE-Wasser) in 30 Minuten zudosiert und 2 Stunden unter unter Eigendruck bei 90°C nachpolymerisiert. Anschließend wurde Zulauf 4 (3.44g tert.-Butylperoctoat, 43.00g Ethanol kosm., 17.00g VE-Wasser) in 30 Minuten zudosiert und nochmals 2 Stunden unter Eigendruck bei 90°C nachpolymerisiert.

### Herstellung von Copolymer gemäß Beispiel 14:

| | | |
|---|---|---|
| Zulauf 1 | 2100,0g | Methylmethacrylat (MMA) |
| | 269,00g | Methacrylsäure (MAS) |
| | 269,00g | Acrylsäure (AS) |
| | 54,00g | Urethanacrylat A (UA A) |
| | 874,00g | Isopropanol |
| | | |
| Zulauf 2 | 270,00g | VE-Wasser (VE = voll entsalzt) |
| | 40,00g | Wako^{®}V-59 |
| | 360,00g | Isopropanol |

Als Vorlage wurden in einem 15-l-Edelstahlreaktor 190,00 g von Zulauf 1 mit 3500,0g Isopropanol und 1075.0g VE-Wasser gemischt. Diese Vorlage wurde mit Stickstoffatmosphäre 3mal abgepresst (5,0bar) und dann bei 0,5bar auf 85°C erhitzt. Danach wurden 20,0g von Zulauf 2 zugegeben. Nach 10 Minuten wurden die Zuläufe 1 und 2 gemeinsam gestartet. Zulauf 1 wurde in 3 Stunden und Zulauf 2 in 4 Stunden bei 85°C unter Eigendruck zudosiert. Die Reaktionsmischung wurde 2 Stunden bei 85°C unter Elgendruck weiter polymerisiert.

2000,0g der erhaltenen Lösung (Feststoffgehalt von 31,5 Gew.-% wurden auf 110°C Temperatur unter Eigendruck erhitzt und Zulauf 3 (1,90g tert,-Butylperoctoat, 25,00g Isopropanol) wurde in 30 Minuten zudosiert und 2 Stunden unter Eigendruck bei 110°C nachpolymerisiert. Anschlieβend wurde Zulauf 4 (1,90g tert,-Butylperoctoat, 26,00g Isopropanol) in 30 Minuten zudosiert und nochmals 2 Stunden unter Eigendruck bei 110°C nachpolymerisiert.

900,00 g der erhaltenen Lösung (Feststoffgehalt von 30,0Gew.∼%) wurden mit 96,0g VE-Wasser verdünnt und mit 41.70g 2-Amino∼2∼Methyi-1-Propanol teilneutralisiert und einer Wasserdampfdestillation unterlegt. Die Mischung wurde danach mit kosm. Ethanol auf einen Feststoffgehalt von ca. 30,8 Gew,-%. Verdünnt. Die resultierende Lösung enthielt ca. 1,3 Gew.-% Isopropanol.

### Herstellung von Copolymer gemäß Beispiel 15:

| | | |
|---|---|---|
| Zulauf 1 | 2100,0g | Methylmethacrylat. (MMA) |
| | 269,00g | Methacrylsäure (MAS) |
| | 269,00g | Acrylsäure (AS) |
| | 54,00g | Urethanacrylat A (UA A) |
| | 874,00g | Isopropanol |
| | 270,00g | VE-Wasser |
| | | |
| Zulauf 2 | | |
| | 40,00g | Wako®V-59 |
| | 360,00g | Isopropanol |

Als Vorlage wurden in einem 15-l-Edelstahlreaktor 190.00 g von Zulauf 1 mit 3500,0g Isopropanol und 1075,0g VE-Wasser gemischt Diese Vorlage wurde mit Stickstoffamosphäre 3mal abgepresst (5,0bar) und dann bei 0,5bar auf 85°C erhitzt. Danach wurden 20,0g von Zulauf 2 zugegeben. Nach 10 Minuten wurden die Zuläufe 1 und 2 gemeinsam gestartet. Zulauf 1 wurde in 3 Stunden und Zulauf 2 in 4 Stunden bei 85°C unter Eigendruck zudosiert. Die Reaktionsmischung wurde 2 Stunden bei 85°C unter Eigendruck weiter polymerisiert.

1815,0g der erhaltenen Lösung wurden in einem 5-l-Edelstahlreaktor mit 355,0g VE Wasser verdünnt und auf 90°C Temperatur unter Elgendruck erhitzt und Zulauf 3 (1,70g Natriumperoxodisufat, 25,0g VE-Wasser) wurde in 30 Minuten zudosiert und 2 Stunden unter Elgendruck bei 90°C nachpolymerisiert. Anschließend wurde Zulauf 4 (1,70g Natriumperoxodisulfat, 25,0g VE-Wasser) in 30 Minuten zudosiert und nochmals 2 Stunden unter Eigendruck bei 90°C nachpolymerisiert.

900,00 g der erhaltenen Lösung (Feststoffgehalt von 30,0Gew,-%) wurden mit 96.0g VE Wasser verdünnt, mit 41,70g 2-Amino-2-Methyl-1-Propanol teilneutralisiert und einer Wasserdampfdestillation unterlegt. Die Mischung wurde danach mit kosm. Ethanol auf einen Feststoffgehalt von ca. 29,7 Gew,-% verdünnt. Die geruchlose Lösung enthielt 0.4% Isopropanol.

### Herstellung von Copolymer gemäß Beispiel 16

| | | |
|---|---|---|
| Zulauf 1 | 234,0g | Methylmethacrylat |
| | 30,00g | Methacrylsäure |
| | 30,00g | Acrylsäure |
| | 100,00g | Ethanol kosm. |
| | 6,00g | Urethanacrylat A |
| | | |
| Zulauf 2 | 6,0g | Wako^{®}V-59 |
| | 412,50g | Ethanol kosm. |
| | 68,40g | VE-Wasser |

Als Vorlage wurden in einem 2-l-Glasreaktor 15,0 g von Zulauf 1 und 24,3g von Zulauf 2 mit 171,50 g kosmetischem Ethanol und 28,60g VE-Wasser gemischt. Diese Vorlage wurde unter Stickstoffatmosphäre zum Rückfluss erhitzt. Nach Erreichen der Rückflusstemperatur wurden die Zuläufe 1 und 2 gemeinsam gestartet. Zulauf 1 wurde in 3 Stunden unter Rückfluss und Zulauf 2 in 4 Stunden unter Rückfluss zudosiert. Die Reaktionsmischung wurde 2 Stunden unter Rückfluss weiter polymerisiert. Danach wurde Zulauf 3 (1,50 g tert.-Butylperpivalat, 3,0 g VE-Wasser und 17,0 g Ethanol kosm.) in 30 Minuten zudosiert und 2 Stunden unter Rückfluss nachpolymerisiert. Anschließend wurde Zulauf 4 (1,50 g tert.-Butylperpivalat, 3,0 g VE-Wasser und 17,0 g Ethanol kosm.) in 30 Minuten zudosiert und nochmals 2 Stunden unter Rückfluss nachpolymerisiert.

### Herstellung von Copolymer gemäß Beispiel 17

| | | |
|---|---|---|
| Zulauf 1 | 234,0g | Methylmethacrylat |
| | 30,00g | Methacrylsäure |
| | 30,00g | Acrylsäure |
| | 6,00g | Urethanacrylat A |
| | | |
| Zulauf 2 | 6,0g | Wako^{®}V-59 |
| | 412,50g | Ethanol kosm. |

Als Vorlage wurden in einem 2-l-Glasreaktor 15,0 g von Zulauf 1 und 21,0 g von Zulauf 2 mit 171,50 g kosmetischen Ethanol und 103,0 g VE-Wasser gemischt. Diese Vorlage wurde unter Stickstoffatmosphäre zum Rückfluss erhitzt. Nach Erreichen der Rückflusstemperatur wurden die Zuläufe 1 und 2 gemeinsam gestartet. Zulauf 1 wurde in 3 Stunden unter Rückfluss und Zulauf 2 in 4 Stunden unter Rückfluss zudosiert. Die Reaktionsmischung wurde 2 Stunden unter Rückfluss weiter polymerisiert. Danach wurde Zulauf 3 (1,50 g tert.-Butylperpivalat und 17,0 g Ethanol kosm.) in 30 Minuten zudosiert und 2 Stunden unter Rückfluss nachpolymerisiert. Anschließend wurde Zulauf 4 (1,50 g tert.-Butylperpivalat und 17,0 g Ethanol kosm.) in 30 Minuten zudosiert und nochmals 2 Stunden unter Rückfluss nachpolymerisiert.

### Herstellung von Copolymer gemäß Beispiel 18

| | | | |
|---|---|---|---|
| Zulauf 1 | | | |
| | 2100,0g | Methylmethacrylat | (MMA) |
| | 269,00g | Methacrylsäure | (MAS) |
| | 269,00g | Acrylsäure | (AS) |
| | 54,00g | Urethanacrylat A | (UA A) |
| | 640,00g | Isopropanol | |
| | 500,00g | VE-Wasser | |
| | | | |
| Zulauf 2 | | | |
| | 40,00g | Wako^{®}V-59 | |
| | 360,00g | Isopropanol | |

Als Vorlage wurden in einem 15-l-Edelstahlreaktor 190,00 g von Zulauf 1 mit 3300,0 g Isopropanol und 1300,00g VE-Wasser gemischt. Diese Vorlage wurde mit Stickstoffamosphäre 3mal abgepresst (5,0bar) und dann bei 0,5bar auf 85°C erhitzt. Danach wurden 20,0g von Zulauf 2 zugegeben. Nach 10 Minuten wurden die Zuläufe 1 und 2 gemeinsam gestartet. Zulauf 1 wurde in 3 Stunden und Zulauf 2 in 4 Stunden bei 80°C unter Eigendruck zudosiert. Die Reaktionsmischung wurde 2 Stunden bei 85°C unter Eigendruck weiter polymerisiert.

1800,00 g der erhaltenen Lösung (Feststoffgehalt von 31,5Gew.-%) wurde mit 280.00g VE-Wasser verdünnt. Danach wurde die Temperatur unter Eigendruck auf 105°C erhöht und Zulauf 3 (1,70g Natriumperoxodisulfat, 22,50g VE-Wasser) wurde in 30 Minuten zudosiert und 2 Stunden unter Eigendruck bei 105°C nachpolymerisiert. Anschließend wurde Zulauf 4 (1,70g Natriumperoxodisulfat, 22,50g VE-Wasser) in 30 Minuten zudosiert und nochmals 2 Stunden unter Eigendruck bei 105°C nachpolymerisiert. 900,00 g der erhaltenen Lösung (Feststoffgehalt von 30,0Gew.-%) wurden mit 41,70g 2-amino-2-methyl-1-propanol teilneutralisiert und einer Wasserdampfdestillation unterlegt. Die Mischung wurde danach mit Ethanol kosm. Verdünnt auf Feststoffgehalt 25,0%. Die geruchlose Lösung enthielt 0,5 Gew.-% Isopropanol.

### Herstellung von Copolymer gemäß Beispiel 19

| | | |
|---|---|---|
| Zulauf 1 | | |
| | 891.6g | Methylmethacrylat |
| | 114.3g | Methacrylsäure |
| | 114.3g | Acrylsäure |
| | 22.9g | Urethanacrylat A |
| | 272.0g | iso-Propanol |
| | 307.8g | VE-Wasser |
| | | |
| Zulauf 2 | | |
| | 14.3g | Wako V 59 |
| | 103.0g | iso-Propanol |

Als Vorlage wurden in einem 15-l-Edelstahlreaktor 86.1g von Zulauf 1 mit 1018.3g l-sopropanol und 702.5g VE-Wasser gemischt. Diese Vorlage wurde mit Stickstoffamosphäre 3mal abgepresst (5,0bar) und dann bei 0,5bar auf 85°C erhitzt. Danach wurden 5.9 g von Zulauf 2 zugegeben. Nach 10 Minuten wurden die Zuläufe 1 und 2 gemeinsam gestartet. Zulauf 1 wurde in 3 Stunden und Zulauf 2 in 4 Stunden bei 85°C unter Eigendruck zudosiert. Die Reaktionsmischung wurde 2 Stunden bei 85°C unter Eigendruck weiter polymerisiert.

Danach wurden 134.9 g VE-Wasser zugegeben und die Temperatur unter Eigendruck auf 110°C erhöht. Zulauf 3 (3.4 g Natriumperoxodisulfat, 44.6 g VE-Wasser) wurde in 45 Minuten zudosiert und 2 Stunden unter Eigendruck bei 110°C nachpolymerisiert. Anschließend wurde Zulauf 4 (3.4 g Natriumperoxodisulfat, 44.6 g VE-Wasser) in 45 Minuten zudosiert und nochmals 2 Stunden unter Eigendruck bei 110°C nachpolymerisiert.

Die erhaltene Lösung wurde mit 70,0g 2-amino-2-methyl-1-propanol (95%) teilneutralisiert und mit 505 g Wasser verdünnt, danach wird die Mischung einer Wasserdampf-destillation unterlegt. Die Mischung wurde danach mit 105,8g 2-amino-2-methyl-1-propanol (95%) teilneutralisiert und mit Ethanol kosm. und Wasser verdünnt auf Feststoffgehalt 34,3 Gew.-%.

### Bestimmung des K-Wertes

Die K-Werte der Copolymere wurden nach Fikentscher, Cellulosechemie, Bd. 13, S. 58 bis 64 (1932) bei 25°C in N-Methylpyrrolidon-(NMP)- oder Ethanol-Lösung gemessen und stellen ein Maß für das Molgewicht dar. Die jeweiligen Lösungen der Polymere enthielten je 1 g Polymer in 100 ml Lösung. Die Messung erfolgte in einer Micro-Ubbelohde-Kapillare Typ M lc der Fa. Schott.

| 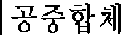 | t-BA | EMA | MMA | SMA | MAA | AA | VP | NtB-EMA | DMA EMA | UDA | Si-UA | 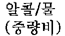 | 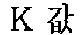 |
|---|---|---|---|---|---|---|---|---|---|---|---|---|---|
| 1 | 75 | | | | 20 | 5 | | | | | | EtOH: H₂O 2.5:1 1 | 37.4 |
| 2 | | 75 | | | 15 | 10 | | | | | | EtOH: H₂O 2.5 : 1 | 35.6 |
| 3 | 72 | | | 3 | 25 | | | | | | | EtOH: H₂O 3.5 : 1 | 36.1 |
| 4 | | | 73 | | 12 | 12 | | | 3 | | | iPrOH: H₂O 2.7 : 1 | 32.5 |
| 5 | 52 | | | | 3 | | 27 | | 18 | | | EtOH:H₂O 2.5 : 1 | 36.6 |
| 6 | 50 | | | | 18 | - | 27 | 5 | | | | EtOH: H₂O 2.5 : 1 | 36.9 |
| 7 | 50 | | | | | | 30 | | 20 | | | EtOH: H₂O 2.5 : 1 | 38.6 |
| 8 | | | 74 | 74 12 | 12 | 12 | | | | 2* | | iPrOH: H₂O 2.7 : 1 | 33.3 |
| 9 | 72 | - | - | 1 | 25 | | | | | | 2 | EtOH: H₂O 3.5 : 1 | 37.2 |
| 10 | - | - | 76 | - | 11 | 11 | | | | | 2 | iPrOH: H₂O 2.7 : 1 | 32.9 |
| 11 | | | 74 | | 12 | 12 | | | | 2* | | EtOH: H₂O 1.3: 1 | 42.5 |
| 12 | - | - | 76 | - | 11 | 11 | | | | | 2 | EtOH:H₂O 1.3 : 1 | 44.7 |
| 13 | - | - | 73 | | 20 | 5 | | | | 2* | | EtOH: H₂O 2.5: 1 | 36.6 |
| 14 | - | - | 78 | | 10 | 10 | | | | 2** | | iPrOH: H₂O [1]: 3.5:1 [2]: 1.2:1 | 33.1 |
| 15 | - | - | 78 | - | 10 | 10 | | | | 2** | | iPrOH: H₂O [1]:3.5:1 [2]: 1.4:1 | 33.1 |
| 16 | - | - | 78 | - | 10 | 10 | | | | 2** | | EtOH:H₂O 5.5:1 | |
| 17 | - | - | 78 | - | 10 | 10 | | | | 2** | | EtOH:H₂O 5.3:1 | |

| Copolymer | t-BA | EMA | MMA | SMA | MAS | AS | VP | NtB-EMA | DMA EMA | UDA | SI-UA | Alkohol-Wasser (Gew,=Verh.) | K-Wert |
|---|---|---|---|---|---|---|---|---|---|---|---|---|---|
| 18 | - | - | 78 | - | 10 | 10 | | | | 2** | | iPrOH: H₂O | 34,7 |
| | | | | | | | | | | | | [1]; 2,5 : 1 | |
| | | | | | | | | | | | | [2]: 1,2:1 | |
| 19 | | | 78 | - | 10 | 10 | | | | 2** | | iPrOH: H₂O | 31,0**** |
| | | | | | | | | | | | | [1]: 1,3 : 1 | |
| | | | | | | | | | | | | [2]: 1,1 : 1 | |

| | | | | | | | | | | | | | |
|---|---|---|---|---|---|---|---|---|---|---|---|---|---|
| * Laromer®UA 19T; ebenfalls geeignete Polymere wurden dadurch erhalten, dass anstelle von Laromer®UA 19T ein Urethandiacrylat aus PEG-1000 (4,5 mol), Neopentylglgkol (1 mol), t-Butylaminoethylmethacrylat (1mol) und lPDl (6mol) (hergestellt analog zu DE 198 38 825) eingesetzt wurde. ** Urethanacrylat A *** Gewichtsverhältnis von Alkohol zu Wasser im Lösungsmittel während der Hauptpolymerisation [1], bzw. der Nachpolymerisation [2] ****K-Wert gemessen in 1 Gew.-%igor ethanolischer Lösung | | | | | | | | | | | | | |

[1] Hauptpolymerisation
[2] Nachpolymerisation

| | | | | | | | | Restmonomergehalt | | |
|---|---|---|---|---|---|---|---|---|---|---|
| Polymer | MMA [%]* | MAS [%]* | AS [%]* | Urethan [%]* | K-Wert | Starter | Wasser [%]* | MMA [mg/kg]** | MAS [mg/kg]** | AS [mg/kg] ** |
| 16 | 78 | 10 | 10 | Urethanacrylat A 2 | | V59[1] /TBPP[2] | 14 [1]/[2] | | | |
| 17 | 78 | 10 | 10 | Urethanacrylat A 2 | | V59[1] /TBPP[2] | 15 [1]/[2] | | | |
| 14 | 78 | 10 | 10 | Urethanacrylat A 2 | 33,3 | V59[1] /TBPO[2] | 15 [1]/[2] | 4 | 20 | 70 |
| 13 | 73 | 20 | 5 | Laromer®UA 19T 2 | 36,6 | TBPO [1]/[2] | 20 [1]/[2] | 190 | 60 | 40 |
| 15 | 78 | 10 | 10 | Urethanacrylat A 2 | 33,3 | V59[1] /NaPS[2] | 15[1] 30[2] | 15 | 20 | 190 |
| 18 | 78 | 10 | 10 | Urethanacrylat A 2 | 34,7 | V59[1] /NaPS[2] | 30[1] 46[2] | = | = | 90 |
| 19 | 78 | 10 | 10 | Urethanacrylat A 2 | 31,0 | V59[1] /NaPS[2] | 42[1] 48 [2] | 3 | 11 | 16 |

| | | | | | | | | | | |
|---|---|---|---|---|---|---|---|---|---|---|
| *[%] Gew.-% ** [mg/kg]: mg Restmonomer pro kg der gesamten Polymerisationsmischung | | | | | | | | | | |

## Patentansprüche

1. Verfahren zur Herstellung von Polymeren, die wenigstens 50 Gew.-% ethylenisch ungesättigter Verbindungen a), ausgewählt aus der Gruppe bestehend aus C₁-C₁₈-Alkyl(meth)acrylaten, einpolymerisiert enthalten, durch radikalische Polymerisation in Lösung, **dadurch gekennzeichnet, dass** wenigstens ein Polymerisationsinitiator ein ethanollöslicher Initiator ist und die Polymerisation in einem Alkohol umfassenden Lösungsmittel durchgeführt wird, das, bezogen auf das Lösungsmittel, 5 bis 50 Gew.-% Wasser enthält und
wobei die Polymerisation der Polymerisationsmischung bis zum Erreichen eines Restmonomergehalts von höchstens 10 Gew.-%, bezogen auf den Feststoffgehalt der Polymerisationsmischung, in Gegenwart wenigstens eines ethanollöslichen Initiators durchgeführt und eine sich anschließende weitere Polymerisation der Polymerisationsmischung in Gegenwart wenigstens eines wasserlöslichen Initiators durchgeführt wird.

2. Verfahren nach Anspruch 1, **dadurch gekennzeichnet, dass** das Lösungsmittel im Bereich von 15 bis 45 Gew.-% Wasser enthält.

3. Verfahren nach einem der Ansprüche1 oder 2, **dadurch gekennzeichnet, dass** das Lösungsmittel im Bereich von 55 bis 85 Gew.-% Alkohol enthält.

4. Verfahren nach einem der Ansprüche 1 bis 3, **dadurch gekennzeichnet, dass** der Alkohol ausgewählt ist aus der Gruppe bestehend aus Ethanol, Isopropanol und Mischungen davon.

5. Verfahren nach einem der Ansprüche 1 bis 4, **dadurch gekennzeichnet, dass**
a) ausgewählt ist aus der Gruppe bestehend aus Methyl(meth)acrylat, Ethyl(meth)acrylat, n-Butyl(meth)acrylat, i-Butyl(meth)acrylat, tert.-Butyl(meth)acrylat und deren Mischungen.

6. Verfahren nach einem der Ansprüche 1 bis 5, **dadurch gekennzeichnet, dass** der ethanollösliche Polymerisationsinitiator ausgewählt ist aus der Gruppe bestehend aus ethanollöslichen Diazo- und Peroxidverbindungen.

7. Verfahren nach einem der Ansprüche 1 bis 6, **dadurch gekennzeichnet, dass** der ethanollösliche Polymerisationsinitiator ausgewählt ist aus der Gruppe bestehend aus Benzoylperoxid, tert.-Amylperoxypivalat, 2,2'-Azobis(4-methoxy-2,4-dimethylvaleronitril), 2,2'-Azobis(2,4-dimethyl valeronitril), Dimethyl 2,2'-azobis(2-methylpropionat), 2,2'-Azobis(2-methylbutyronitril), 1,1'-Azobis(cyclohexan-1-carbonitril), 2,2'-Azobis[N-(2-propenyl)-2-methylpropionamid], 1-[(cyano-1-methylethyl)azo] formamid, 2,2'-Azobis(N-butyl-2-methylpropionamid), 2,2'-Azobis(N-cyclohexyl-2-methylpropionamid).

8. Verfahren nach einem der Ansprüche 1 bis 7, **dadurch gekennzeichnet, dass** die Temperatur, bei der die Polymerisation durchgeführt wird, im Bereich von 30 bis 120°C liegt.

9. Verfahren nach einem der Ansprüche 1 bis 8, **dadurch gekennzeichnet, dass** der als Lösungsmittel eingesetzte Alkohol nach der Polymerisation weitestgehend durch Destillation entfernt wird.

10. Verfahren nach Anspruch 9, **dadurch gekennzeichnet, dass** das Polymer vor der Destillation in einem Bereich von 20% bis 100% neutralisiert wird.

## Claims

1. A method of producing polymers which comprise, in copolymerized form, at least 50% by weight of ethylenically unsaturated compounds a) chosen from the group consisting of C₁-C₁₈-alkyl (meth)acrylates, by free-radical polymerization in solution, wherein at least one polymerization initiator is an ethanol-soluble initiator, and the polymerization is carried out in an alcohol-comprising solvent which, based on the solvent, comprises 5 to 50% by weight of water and where the polymerization of the polymerization mixture is carried out until a residual monomer content of at most 10% by weight is achieved, based on the solids content of the polymerization mixture, in the presence of at least one ethanol-soluble initiator, and a subsequent further polymerization of the polymerization mixture is carried out in the presence of at least one water-soluble initiator.

2. The method according to claim 1, wherein the solvent comprises water in the range from 15 to 45% by weight.

3. The method according to one of claims 1 or 2,
wherein the solvent comprises alcohol in the range from 55 to 85% by weight.

4. The method according to one of claims 1 to 3, wherein the alcohol is chosen from the group consisting of ethanol, isopropanol and mixtures thereof.

5. The method according to one of claims 1 to 4, wherein a) is chosen from the group consisting of methyl (meth)acrylate, ethyl (meth)acrylate, n-butyl (meth)acrylate, isobutyl (meth)acrylate, tert-butyl (meth)acrylate and mixtures thereof.

6. The method according to one of claims 1 to 5, wherein the ethanol-soluble polymerization initiator is chosen from the group consisting of ethanol-soluble diazo and peroxide compounds.

7. The method according to one of claims 1 to 6, wherein the ethanol-soluble polymerization initiator is chosen from the group consisting of benzoyl peroxide, tert-amyl peroxipivalate, 2,2'-azobis(4-methoxy-2,4-dimethylvaleronitrile), 2,2'-azobis(2,4-dimethylvaleronitrile), dimethyl 2,2'-azobis(2-methylpropionate), 2,2'-azobis(2-methylbutyronitrile), 1,1'-azobis(cyclohexane-1-carbonitrile), 2,2'-azobis[N-(2-propenyl)-2-methylpropionamide], 1-[(cyano-1-methylethyl)azo] formamide, 2,2'-azobis(N-butyl-2-methylpropionamide), 2,2'-azobis(N-cyclohexyl-2-methylpropionamide).

8. The method according to one of claims 1 to 7, wherein the temperature at which the polymerization is carried out is in the range from 30 to 120°C.

9. The method according to one of claims 1 to 8, wherein, following the polymerization, the alcohol used as solvent is removed by distillation to the greatest possible extent.

10. The method according to claim 9, wherein the polymer is neutralized in a range from 20% to 100% before the distillation.

## Revendications

1. Procédé de fabrication de polymères qui contiennent sous forme polymérisée au moins 50 % en poids de composés éthyléniquement insaturés a), choisis dans le groupe constitué par les (méth)acrylates d'alkyle en C₁-C₁₈, par polymérisation radicalaire en solution, **caractérisé en ce qu'**au moins un initiateur de polymérisation est un initiateur soluble dans l'éthanol et la polymérisation est réalisée dans un solvant comprenant un alcool qui contient, par rapport au solvant, 5 à 50 % en poids d'eau, la polymérisation du mélange de polymérisation étant réalisée en présence d'au moins un initiateur soluble dans l'éthanol jusqu'à atteindre une teneur résiduelle en monomères d'au plus 10 % en poids, par rapport à la teneur en solides du mélange de polymérisation, et une polymérisation supplémentaire ultérieure du mélange de polymérisation étant réalisée en présence d'au moins un initiateur soluble dans l'eau.

2. Procédé selon la revendication 1, **caractérisé en ce que** le solvant contient dans la plage allant de 15 à 45 % en poids d'eau.

3. Procédé selon l'une quelconque des revendications 1 ou 2, **caractérisé en ce que** le solvant contient dans la plage allant de 55 à 85 % en poids d'un alcool.

4. Procédé selon l'une quelconque des revendications 1 à 3, **caractérisé en ce que** l'alcool est choisi dans le groupe constitué par l'éthanol, l'isopropanol et leurs mélanges.

5. Procédé selon l'une quelconque des revendications 1 à 4, **caractérisé en ce que** a) est choisi dans le groupe constitué par le (méth)acrylate de méthyle, le (méth)acrylate d'éthyle, le (méth)acrylate de n-butyle, le (méth)acrylate d'i-butyle, le (méth)acrylate de tert.-butyle et leurs mélanges.

6. Procédé selon l'une quelconque des revendications 1 à 5, **caractérisé en ce que** l'initiateur de polymérisation soluble dans l'éthanol est choisi dans le groupe constitué par les composés diazoïques et peroxydiques solubles dans l'éthanol.

7. Procédé selon l'une quelconque des revendications 1 à 6, **caractérisé en ce que** l'initiateur de polymérisation soluble dans l'éthanol est choisi dans le groupe constitué par le peroxyde de benzoyle, le peroxypivalate de tert.-amyle, le 2,2'-azobis(4-méthoxy-2,4-diméthylvaléronitrile), le 2,2'-azobis(2,4-diméthylvaléronitrile), le 2,2'-azobis(2-méthylpropionate) de diméthyle, le 2,2'-azobis(2-méthylbutyronitrile), le 1,1'-azobis(cyclohexane-1-carbonitrile), le 2,2'-azobis[N-(2-propényl)-2-méthylpropionamide], le 1-[(cyano-1-méthyléthyl)azo]formamide, le 2,2'-azobis(N-butyl-2-méthylpropionamide), le 2,2'-azobis(N-cyclohexyl-2-méthylpropionamide).

8. Procédé selon l'une quelconque des revendications 1 à 7, **caractérisé en ce que** la température à laquelle la polymérisation est réalisée se situe dans la plage allant de 30 à 120 °C.

9. Procédé selon l'une quelconque des revendications 1 à 8, **caractérisé en ce que** l'alcool utilisé en tant que solvant est éliminé autant que possible par distillation après la polymérisation.

10. Procédé selon la revendication 9, **caractérisé en ce que** le polymère est neutralisé avant la distillation dans une plage allant de 20 % à 100 %.
